(19) Europäisches Patentamt
European Patent Office
Office européen des brevets

(11) **EP 1 915 137 B1**

(12) **EUROPÄISCHE PATENTSCHRIFT**

(45) Veröffentlichungstag und Bekanntmachung des Hinweises auf die Patenterteilung:
**07.08.2013 Patentblatt 2013/32**

(21) Anmeldenummer: 05774025.0

(22) Anmeldetag: **10.08.2005**

(51) Int Cl.:
***A61K 9/50*** *(2006.01)*          ***A61K 9/16*** *(2006.01)*

(86) Internationale Anmeldenummer:
**PCT/EP2005/008700**

(87) Internationale Veröffentlichungsnummer:
**WO 2007/016948 (15.02.2007 Gazette 2007/07)**

(54) **ORALES PRÄPARAT MIT KONTROLLIERTER FREISETZUNG**

ORAL PREPARATION WITH CONTROLLED RELEASE

PRÉPARATION ORALE À LIBÉRATION CONTRÔLÉE

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**30.04.2008 Patentblatt 2008/18**

(73) Patentinhaber: **ADD Advanced Drug Delivery Technologies, Ltd.**
**4153 Reinach (CH)**

(72) Erfinder:
• **SCHLÜTERMANN, Burkhard**
**79280 Au (DE)**

• **KOHLMEYER, Manfred**
**CH-4053 Basel (CH)**

(74) Vertreter: **Henkel, Breuer & Partner**
**Patentanwälte**
**Erika-Mann Strasse 23**
**80636 München (DE)**

(56) Entgegenhaltungen:
**EP-A- 0 609 961     WO-A-01/15668**
**US-A- 5 955 104     US-A1- 2003 165 565**

**Beschreibung**

**Gebiet der Erfindung**

Die vorliegende Erfindung betrifft neue pharmazeutische Pellets, multipartikuläre Darreichungsformen auf der Basis solcher Pellets, Verfahren zur Herstellung von Pellets sowie Verfahren zur Herstellung von Darreichungsformen unter Verwendung der Pellets. Die Pellets und die multipartikulären Darreichungsformen auf der Basis der Pellets zeichnen sich insbesondere durch eine gesteuerte Wirkstofffreisetzung aus.

**Hintergrund der Erfindung**

Bei oraler Verabreichung von Arzneimitteln wird der Wirkstoff im Magen-DarmTrakt freigesetzt, und ein Anteil des Wirkstoffs wird resorbiert. Durch Steuerung der Freisetzung des Wirkstoffs können das Ausmaß der Resorption und die Wirkungsdauer beeinflusst werden. Entsprechend sind verschiedene Vorschläge gemacht worden, um die Wirkstofffreisetzung durch geeignete galenische Formulierung des Wirkstoffs zu steuern.

Ein Ansatz besteht darin, Darreichungsformen mit Überzügen zu versehen, wobei die Wirkstofffreisetzung abhängig von der Löslichkeit oder Durchlässigkeit der Überzüge beeinflusst werden kann. Derartige Überzüge können beispielsweise auf Tabletten oder Kapseln aufgebracht werden. In diesem Fall besteht jedoch ein Nachteil darin, dass ein fehlerhafter oder beschädigter Überzug dazu führen kann, dass die Freisetzung der gesamten Wirkstoffdosis nicht in der gewünschten Weise gesteuert wird.

Als Alternative bieten sich multipartikuläre Darreichungsformen an, bei denen die Gesamtmenge des Wirkstoffs auf eine größere Anzahl kleinerer Einheiten, wie Pellets, verteilt ist. Werden die einzelnen Pellets mit Überzügen versehen, so unterliegt im Fall eines fehlerhaften Überzugs bei einem Pellet nur ein entsprechend geringer Anteil der gesamten Wirkstoffdosis nicht der gewünschten Freisetzung.

Ein weiterer Vorteil derartiger Darreichungsformen auf der Basis von Pellets besteht darin, dass hinreichend kleine Pellets nach Einnahme relativ rasch aus dem Magen in den Darm übertreten. Tabletten hingegen können, sofern sie nicht zerfallen, auch für längere Zeit im Magen verbleiben, wobei die Zeit zudem recht variabel ist.

Ungeachtet der bekannten Vorteile von Pellets bzw. multipartikulären Darreichungsformen ist es jedoch schwierig, ein gewünschtes Freisetzungsverhalten einzustellen. Dies hängt damit zusammen, dass es nach dem Stand der Technik schwierig ist, gleichmäßig überzogene Pellets bereitzustellen. Schon die Pelletkerne, die überzogen werden sollen, weisen eine unzureichende Qualität auf. Insbesondere Pellets, die durch Extrusion hergestellt werden, sind häufig ungleichmäßig in ihrer Form und haben zudem eine raue und unebene Oberfläche, was das anschließende Befilmen schwierig macht und dazu führt, dass Filme guter Qualität kaum erhalten werden können.

Die zur Steuerung der Freisetzung herangezogenen Filme oder Überzüge können verschiedene Zusammensetzungen aufweisen. So sind Vorschläge gemacht worden, die Freisetzung abhängig vom pH-Wert, von der Zeit oder von bakteriellen Enzymen, die im Darm vorhanden sind, zu steuern.

Bei pH-gesteuerten Systemen besteht ein Problem jedoch darin, dass die Wirkstofffreisetzung durch die Nahrungsaufnahme, die den pH-Wert im Magen-Darm-Trakt beeinflusst, verändert wird. Zudem gibt es zwischen verschiedenen Personen erhebliche Unterschiede hinsichtlich der pH-Werte im Magen-DarmTrakt. Auch bei enzymatisch gesteuerten Darreichungsformen mit kontrollierter Freisetzung ist über eine Variabilität berichtet worden.

Bekannte Darreichungsformen mit kontrollierter Freisetzung sind somit nicht völlig zufriedenstellend. Zudem besteht das Problem, dass sich gewünschte (vorgegebene) Freisetzungsprofile nicht einstellen lassen. Ferner ist die Herstellung von Darreichungsformen mit kontrollierter Freisetzung oftmals schwierig. Es besteht somit ein Bedarf an neuen Darreichungsformen mit kontrollierter Freisetzung sowie auch an neuen Verfahren zur Herstellung von Darreichungsformen mit kontrollierter Freisetzung.

**Aufgaben und Kurzdarstellung der Erfindung**

Eine Aufgabe der vorliegenden Erfindung besteht darin, ein pharmazeutisches Pellet bereitzustellen, bei dem die Wirkstofffreisetzung unabhängig vom pH-Wert und unabhängig von der Einwirkung von Enzymen gesteuert werden kann.

Eine weitere Aufgabe besteht darin, ein pharmazeutisches Pellet bereitzustellen, bei dem die Wirkstofffreisetzung einem Profil mit einer Lag-Phase folgt. Weiterhin besteht eine Aufgabe darin, ein Pellet bereitzustellen, bei dem die Wirkstofffreisetzung nach einer Lag-Phase mit einer festgelegten Rate erfolgt. Erfindungsgemäß sollen darüber hinaus Pelletprodukte oder Kollektive von Pellets bereitgestellt werden, die eine Mehrzahl von einzelnen Pellets umfassen, die jeweils den angegebenen Anforderungen genügen. Schließlich sollen erfindungsgemäß Verfahren zur Herstellung von Pellets, Pelletprodukten und anderen Darreichungsformen bereitgestellt werden.

Erfindungsgemäß wurde nun festgestellt, dass Präparate bereitgestellt werden können, bei denen die Wirkstofffreisetzung pH-unabhängig und unabhängig von Enzymen erfolgt, wenn Pellets verwendet werden, die einen kugelförmigen wirkstoffhaltigen Kern mit glatter Oberfläche und einen Überzug auf dem Kern aufweisen, wobei der kern eine mittlere Rauheit von weniger als 10 $\mu$m und vorzugsweise von weniger als 7,5 $\mu$m und/oder eine relative mittlere Rauheit von von weniger als 2% aufweist.

**Kurze Beschreibung der Figuren**

Die Erfindung wird nachstehend unter Bezugnahme auf die Figuren näher erläutert.

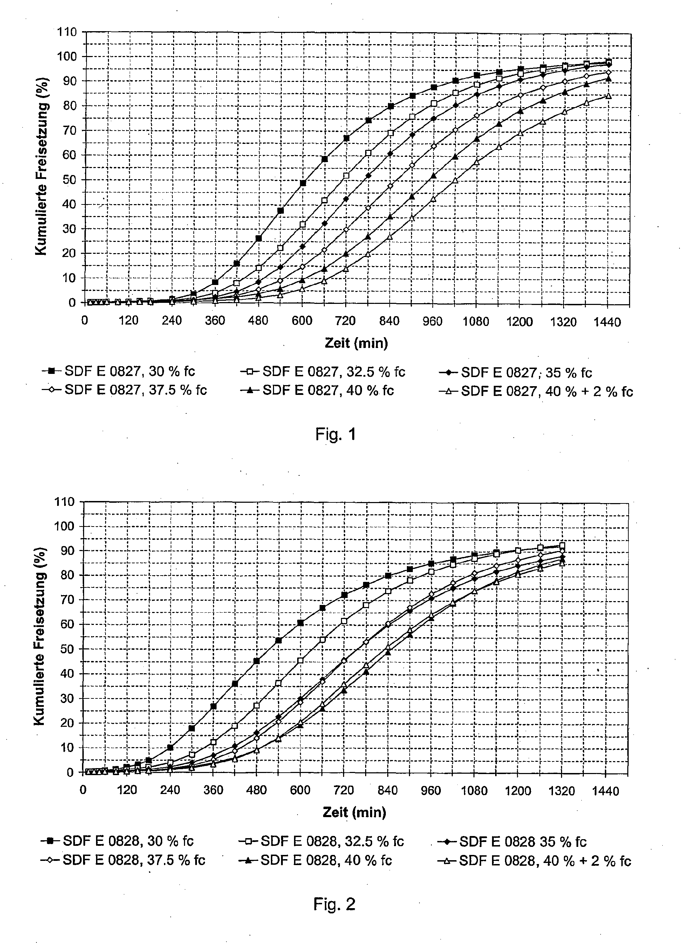

Fig. 1 zeigt den Einfluss der Dicke des die Freisetzung steuernden Überzugs auf die Wirkstofffreisetzung bei Pellets enthaltend den Wirkstoff ADD 006, wobei der Überzug aus Polyvinylacetat und, bezogen auf das Gewicht des Polyvinylacetats, 10 Gew-% Triethylcitrat und 10 Gew.-% Talkum besteht. Aufgetragen ist die kumulative Freisetzung (%) gegen die Zeit (Minuten). Als Maß für die Dicke des Überzugs ist für die einzelnen Chargen die prozentuale Menge an Überzugsmaterial (Polyvinylacetat, Triethylcitrat und Talkum in der vorstehend angegebenen Zusammensetzung), bezogen auf das Gewicht der wirkstoffhaltigen Pelletkerne, angegeben.

Fig. 2 zeigt den Einfluss der Dicke des die Freisetzung steuernden Überzugs auf die Wirkstofffreisetzung bei Pellets enthaltend den Wirkstoff ADD 006, wobei der Überzug aus Polyvinylacetat und, bezogen auf das Gewicht des Polyvinylacetats, 7,5 Gew.-% Triethylcitrat und 10 Gew.-% Talkum besteht. Aufgetragen ist die kumulative Freisetzung (%) gegen die Zeit (Minuten). Als Maß für die Dicke des Überzugs ist für die einzelnen Chargen die prozentuale Menge an Überzugsmaterial (Polyvinylacetat, Triethylcitrat und Talkum in der vorstehend angegebenen Zusammensetzung), bezogen auf das Gewicht der wirkstoffhaltigen Pelletkerne, angegeben.

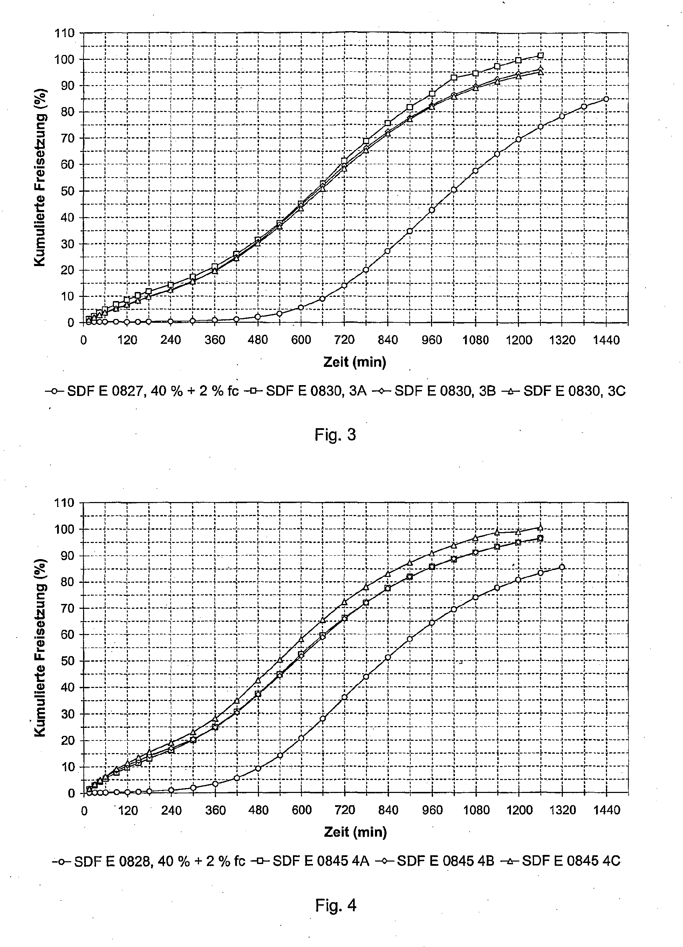

Fig. 3 zeigt den Einfluss der Presskraft auf die Wirkstofffreisetzung aus Tabletten mit einem Durchmesser von 12 mm und einem Gewicht von 884,2 mg, die 200 mg ADD 006 enthalten. Die Tabletten wurden jeweils unter Verwendung von Pellets hergestellt, die einen Pelletkern mit dem Wirkstoff ADD 006 aufweisen. Aufgebracht auf den Pelletkern waren 40 Gew.-% eines Überzugs, bezogen auf das Gewicht der Pelletkerne, aus Polyvinylacetat, Triethylcitrat (10 Gew.-%, bezogen auf das Gewicht des Polyvinylacetats) und Talkum (10 Gew.-%, bezogen auf das Gewicht des Polyvinylacetats) sowie ein Schutzüberzug aus Hydroxypropylmethylcellulose und kolloidalem Siliciumdioxid (2 Gew.-% Schutzüberzug, bezogen auf das Gewicht der Pellets mit dem Polvinylacetat-Überzug). Dargestellt ist die kumulative Freisetzung (%), aufgetragen gegen die Zeit. Die Kurven zeigen die Freisetzung aus den Pellets selbst sowie aus Tabletten, die unter unterschiedlichen Bedingungen hergestellt wurden.

Die Messreihe A betrifft Tabletten, die mit einem Pressdruck von 141 MPa hergestellt wurden und eine Härte von 80 N sowie eine Zerfallszeit kleiner 1 Minute aufweisen. Die Messreihe B betrifft Tabletten, die mit einem Pressdruck von 168 MPa hergestellt wurden und eine Härte von 125 N sowie eine Zerfallszeit von 1 bis 2 Minuten aufweisen. Die Messreihe C betrifft Tabletten, die mit einem Pressdruck von 221 MPa hergestellt wurden und eine Härte von 170 N sowie eine Zerfallszeit von 3 bis 4 Minuten aufweisen.

Fig. 4 zeigt den Einfluss der Presskraft auf die Wirkstofffreisetzung aus Tabletten mit einem Durchmesser von 12 mm und einem Gewicht von 884,2 mg, die 200 mg ADD 006 enthalten. Die Tabletten wurden jeweils unter Verwendung von Pellets hergestellt, die einen Pelletkern mit dem Wirkstoff ADD 006 aufweisen. Aufgebracht auf den Pelletkern waren 40 Gew.-% eines Überzugs, bezogen auf das Gewicht der Pelletkerne, aus Polyvinylacetat, Triethylcitrat (7,5 Gew.-%, bezogen auf das Gewicht des Polyvinylacetats) und Talkum (10 Gew.-%, bezogen auf das Gewicht des Polyvinylacetats) sowie ein Schutzüberzug aus Hydroxypropylmethylcellulose und kolloidalem Siliciumdioxid (2 Gew.-% Schutzüberzug, bezogen auf das Gewicht der Pellets mit dem Polvinylacetat-Überzug). Dargestellt ist die kumulative Freisetzung (%), aufgetragen gegen die Zeit. Die Kurven zeigen die Freisetzung aus den Pellets selbst sowie aus Tabletten, die unter unterschiedlichen Bedingungen hergestellt wurden.

Die Messreihe A betrifft Tabletten, die mit einem Pressdruck von 180 MPa hergestellt wurden und eine Härte von 80 N sowie eine Zerfallszeit kleiner 1 Minute aufweisen. Die Messreihe B betrifft Tabletten, die mit einem Pressdruck von 240 MPa hergestellt wurden und eine Härte von 120 N sowie eine Zerfallszeit kleiner 1 Minute aufweisen. Die Messreihe C betrifft Tabletten, die mit einem Pressdruck von 256 MPa hergestellt wurden und eine Härte von 160 N und eine Zerfallszeit kleiner 1 Minute aufweisen.

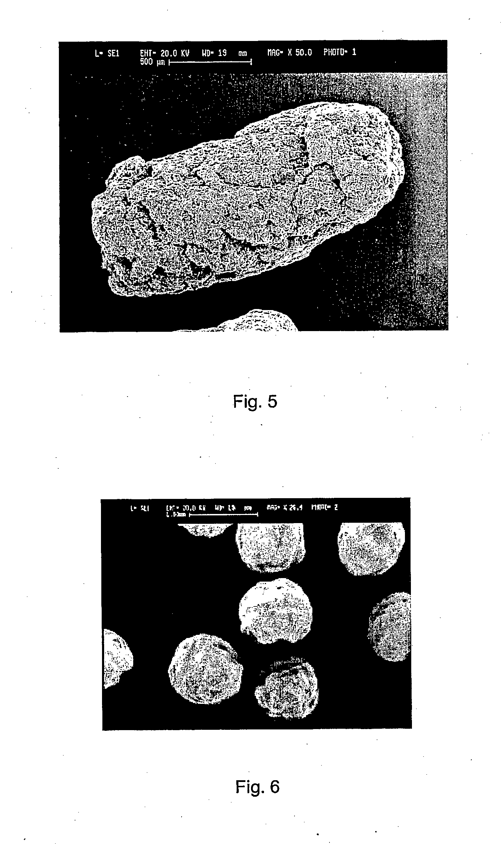

Fig. 5 zeigt eine rasterelektronenmikroskopische Aufnahme (50-fache Vergrößerung) eines Extrusionspellets mit ungleichmäßiger Form und rauer Oberfläche.

Fig. 6 zeigt eine elektronenmikroskopische Aufnahme (25-fache Vergrößerung) von Pellets der Chargenbezeichnung SFD E 0724, die die erfindungsgemäßen Kriterien für einen Pelletkern mit glatter Oberfläche nicht erfüllen.

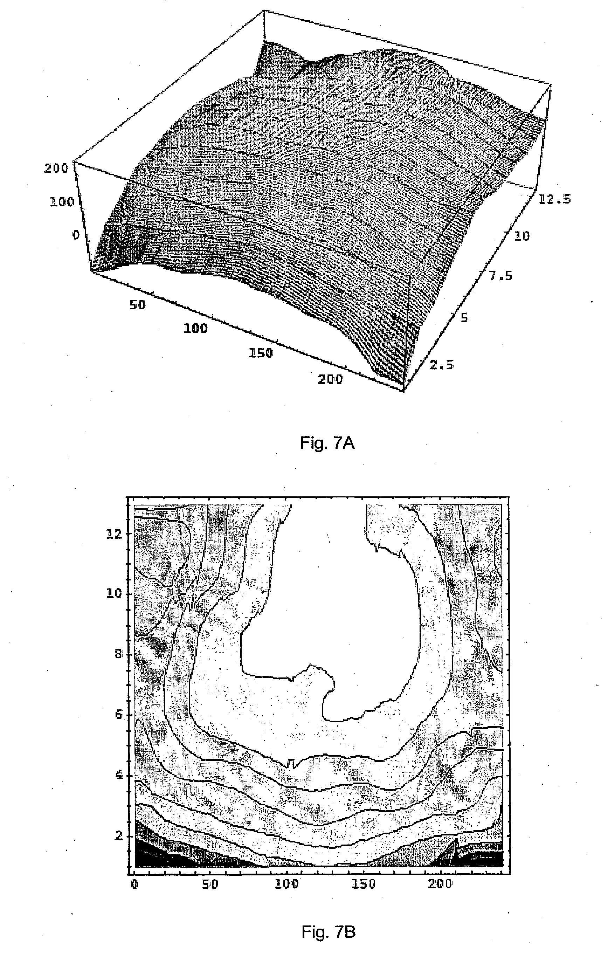

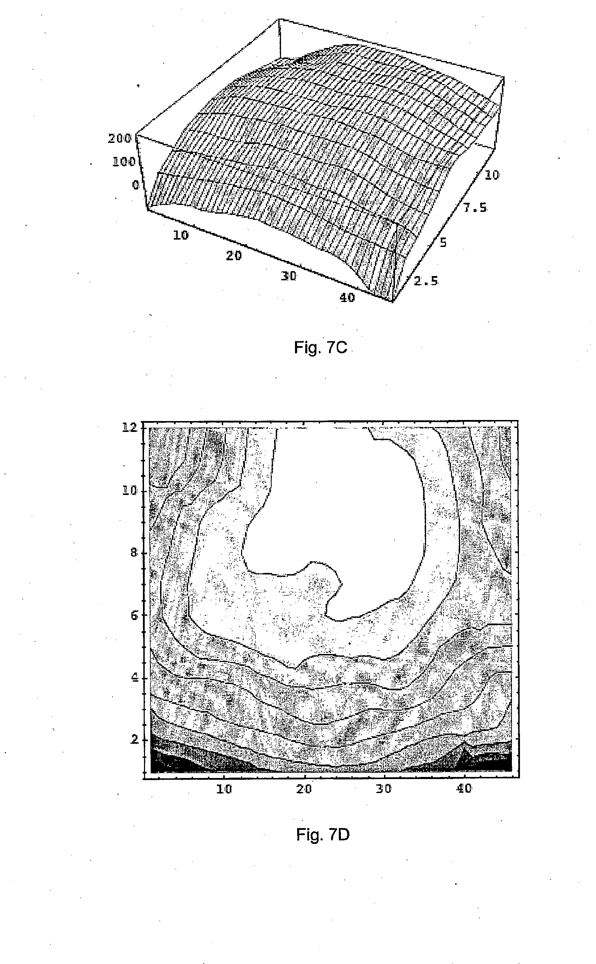

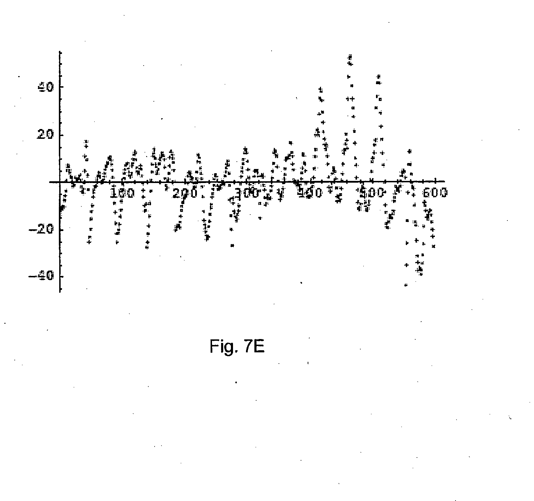

Fig. 7 veranschaulicht durch Abtastung mit einem Laserprofilometer erhaltene Daten, die die Oberfläche eines Pellets der Chargenbezeichnung SFD E 0724 charakterisieren. Dabei ist Fig. 7 A eine grafische Darstellung der Messergebnisse als Oberflächengrafik. In Fig. 7 B sind die Messergebnisse als Höhenliniendiagramm gezeigt. Fig. 7 C zeigt eine Oberflächengrafik auf der Grundlage eines reduzierten Datensatzes. Fig. 7 D zeigt das entsprechende Höhenliniendiagramm. Fig. 7 E zeigt die Abstände der Messpunkte des reduzierten Datensatzes von der nach der Methode der kleinsten Fehlerquadrate ermittelten idealen Oberfläche.

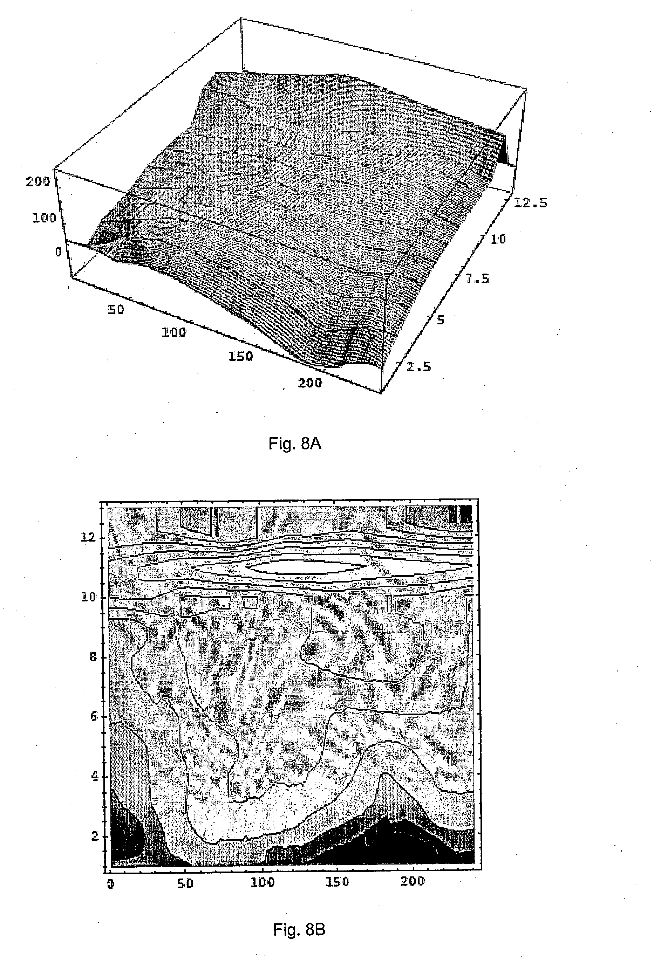

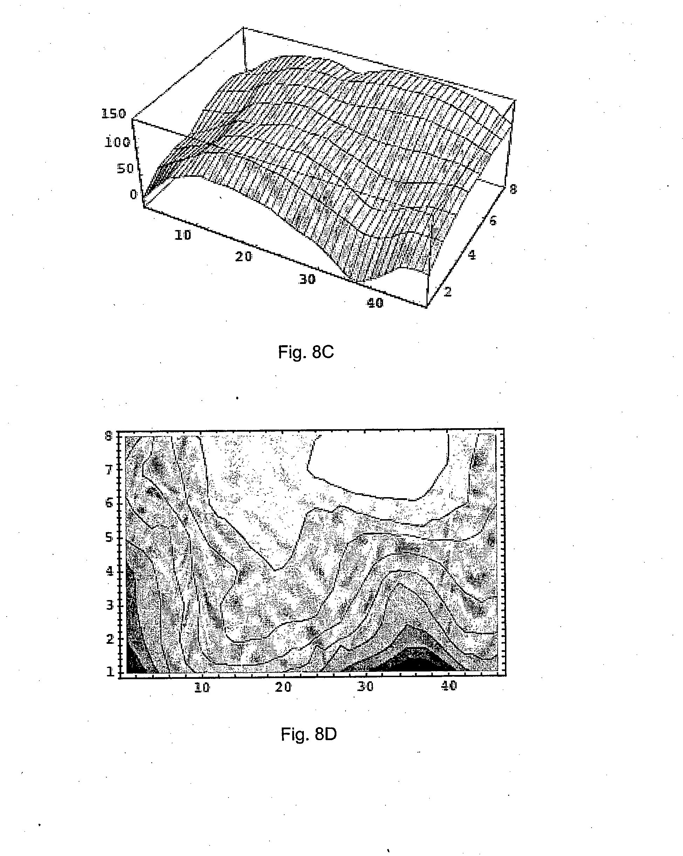

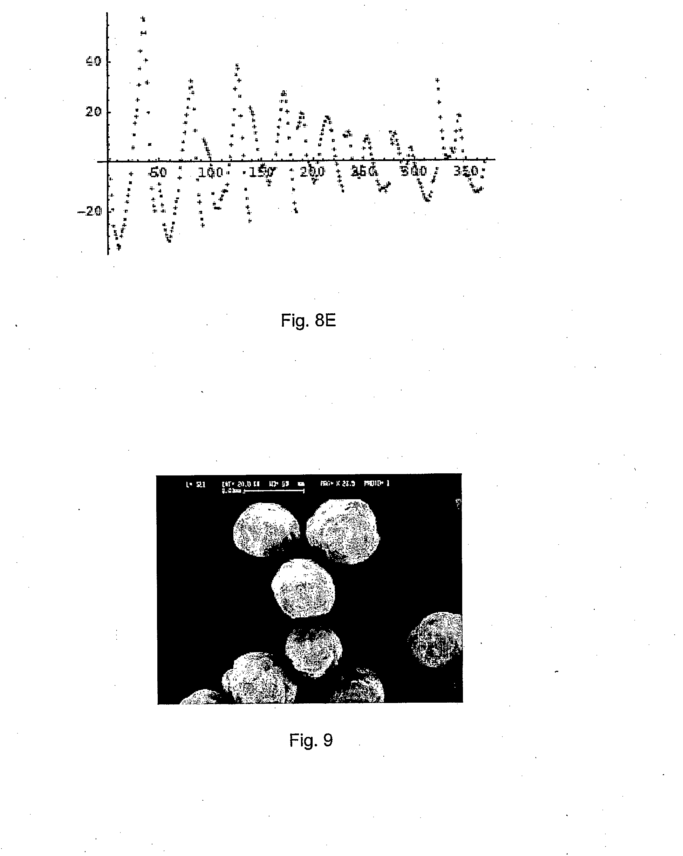

Fig. 8 veranschaulicht durch Abtastung mit einem Laserprofilometer erhaltene Daten, die die Oberfläche eines weiteren Pellets der Chargenbezeichnung SFD E 0724 charakterisieren. Dabei ist Fig. 8 A eine grafische Darstellung der Messergebnisse als Oberflächengrafik. In Fig. 8 B sind die Messergebnisse als Höhenliniendiagramm gezeigt. Fig. 8 C zeigt eine Oberflächengrafik auf der Grundlage eines reduzierten Datensatzes. Fig. 8 D zeigt das entsprechende Höhenliniendiagramm. Fig. 8 E zeigt die Abstände der Messpunkte des reduzierten Datensatzes von der nach der Methode der kleinsten Fehlerquadrate ermittelten idealen Oberfläche.

Fig. 9 zeigt eine elektronenmikroskopische Aufnahme (25-fache Vergrößerung) von Pellets der Chargenbezeichnung SFD E 0718, die die erfindungsgemäßen Kriterien für einen Pelletkern mit glatter Oberfläche nicht erfüllen.

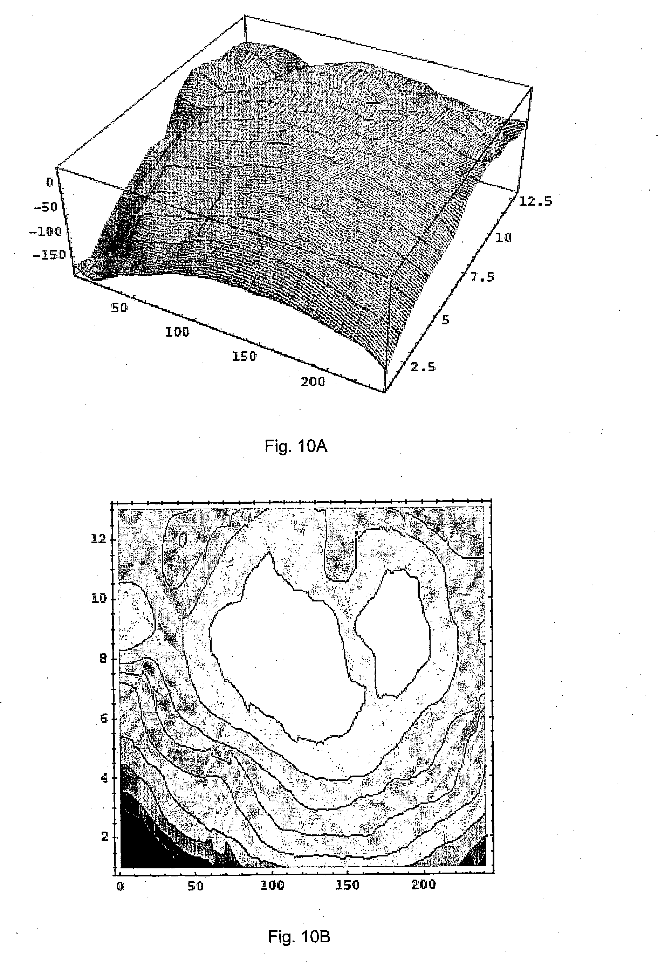

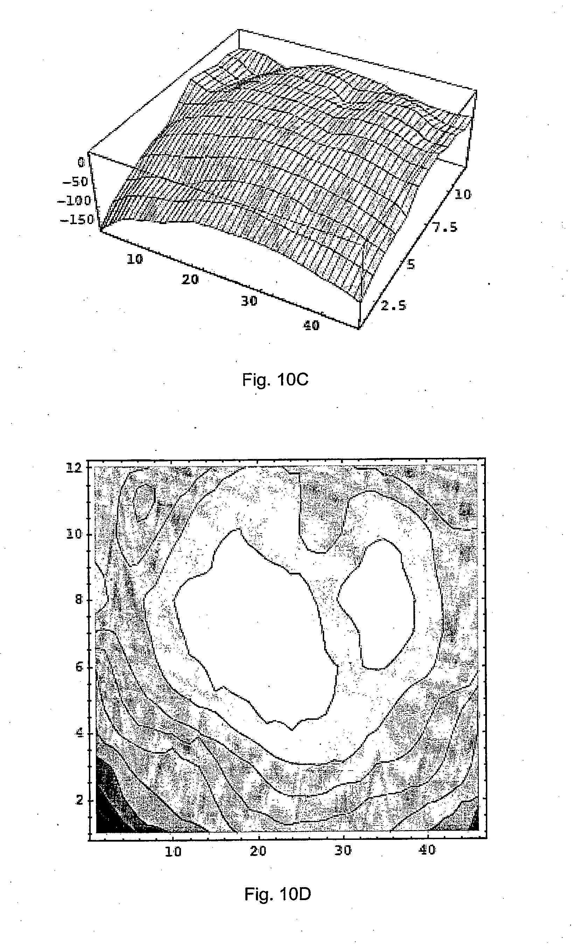

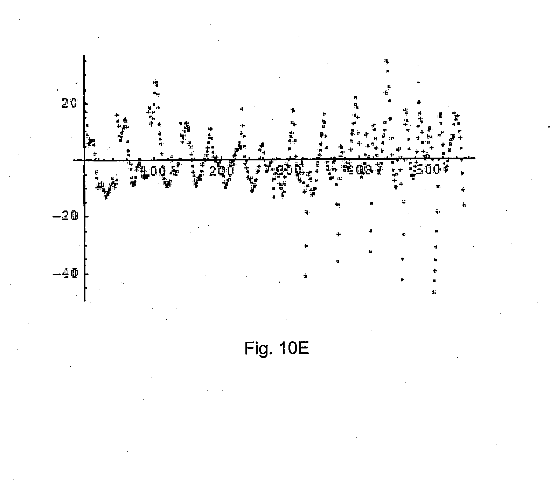

Fig. 10 veranschaulicht durch Abtastung mit einem Laserprofilometer erhaltene Daten, die die Oberfläche eines Pellets der Chargenbezeichnung SFD E 0718 charakterisieren. Dabei ist Fig. 10 A eine grafische Darstellung der Messergebnisse als Oberflächengrafik. In Fig. 10 B sind die Messergebnisse als Höhenliniendiagramm gezeigt. Fig. 10 C zeigt eine Oberflächengrafik auf der Grundlage eines reduzierten Datensatzes. Fig. 10 D zeigt das entsprechende Höhenliniendiagramm. Fig. 10 E zeigt die Abstände der Messpunkte des reduzierten Datensatzes von der nach der Methode der kleinsten Fehlerquadrate ermittelten idealen Oberfläche.

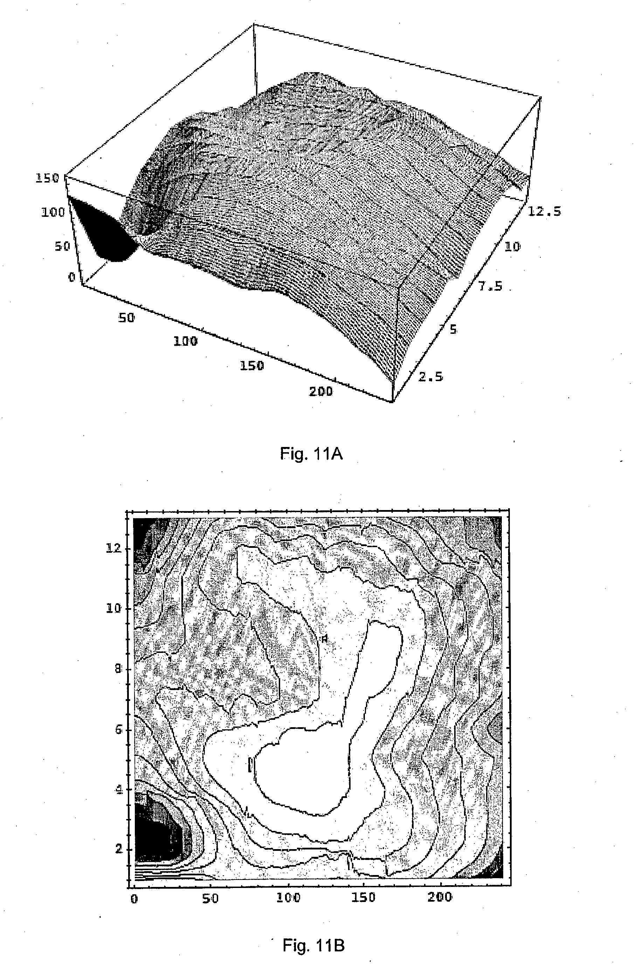

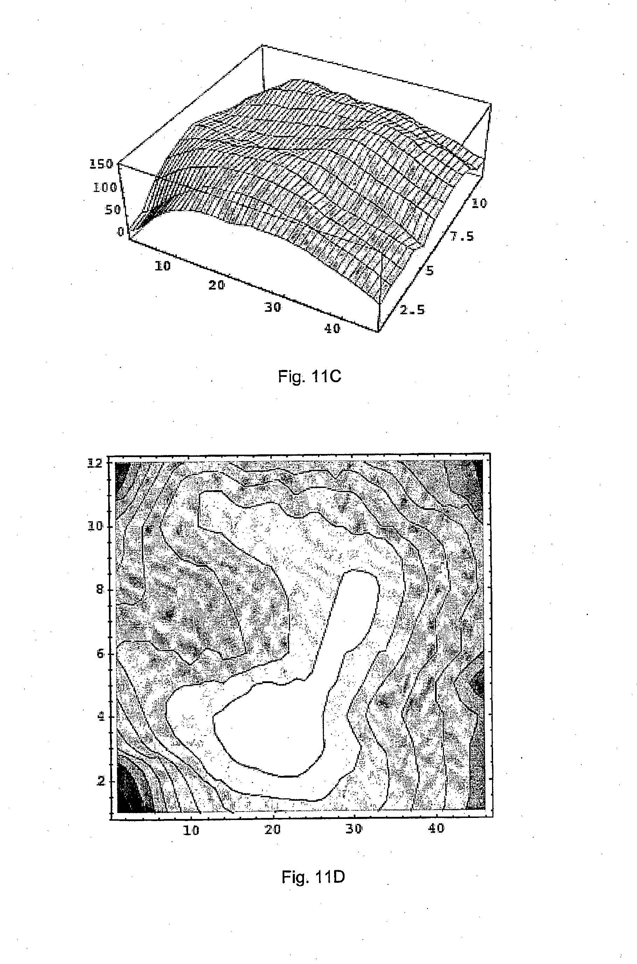

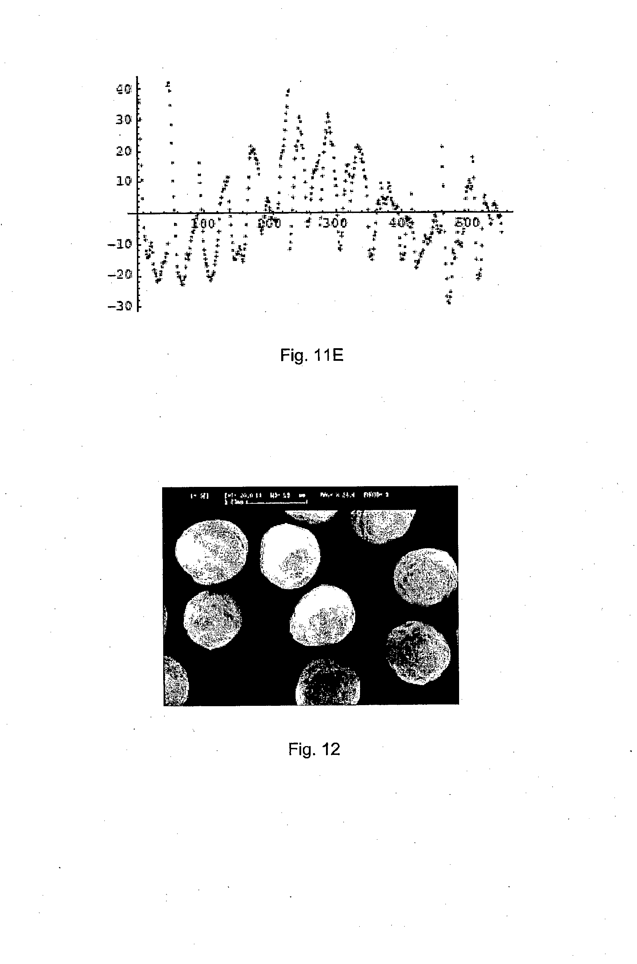

Fig. 11 veranschaulicht durch Abtastung mit einem Laserprofilometer erhaltene Daten, die die Oberfläche eines weiteren Pellets der Chargenbezeichnung SFD E 0718 charakterisieren. Dabei ist Fig. 11 A eine grafische Darstellung der Messergebnisse als Oberflächengrafik. In Fig. 11 B sind die Messergebnisse als Höhenliniendiagramm gezeigt. Fig. 11 C zeigt eine Oberflächengrafik auf der Grundlage eines reduzierten Datensatzes. Fig. 11 D zeigt das entsprechende Höhenliniendiagramm. Fig. 11 E zeigt die Abstände der Messpunkte des reduzierten Datensatzes von der nach der Methode der kleinsten Fehlerquadrate ermittelten idealen Oberfläche.

Fig. 12 zeigt eine elektronenmikroskopische Aufnahme (25-fache Vergrößerung) von Pellets der Chargenbezeichnung SFD E 0572, die die erfindungsgemäßen Kriterien für einen Pelletkern mit glatter Oberfläche nicht erfüllen.

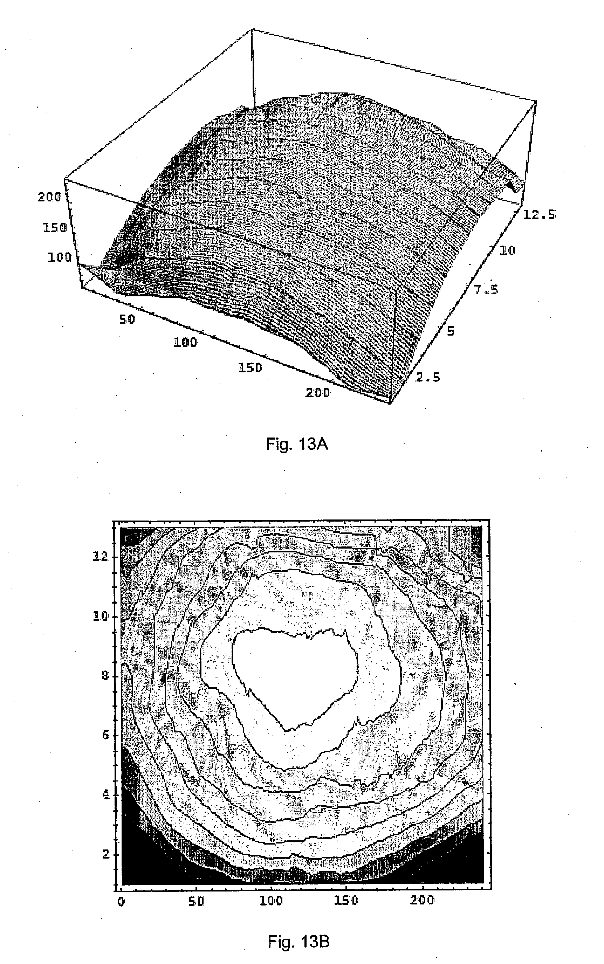

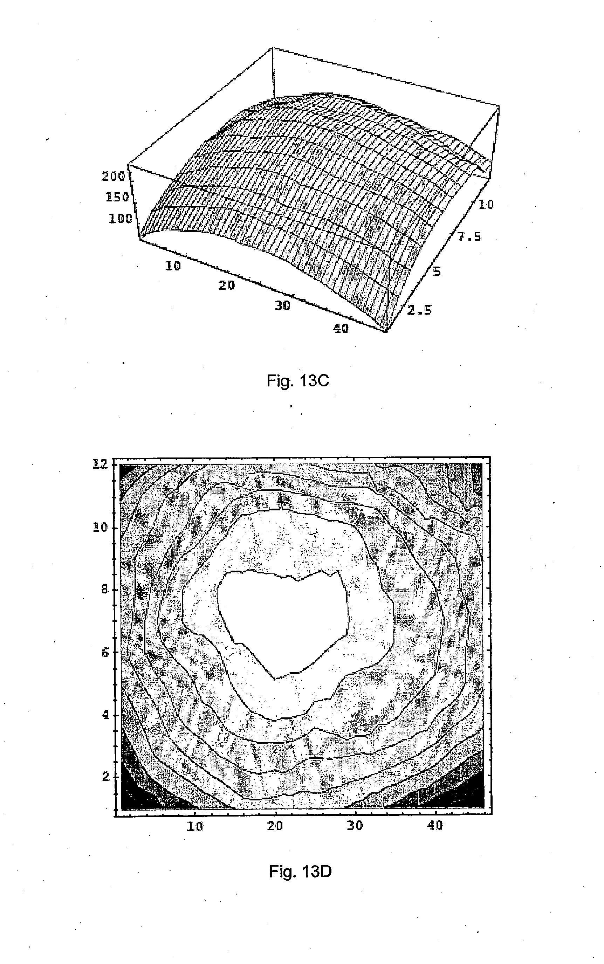

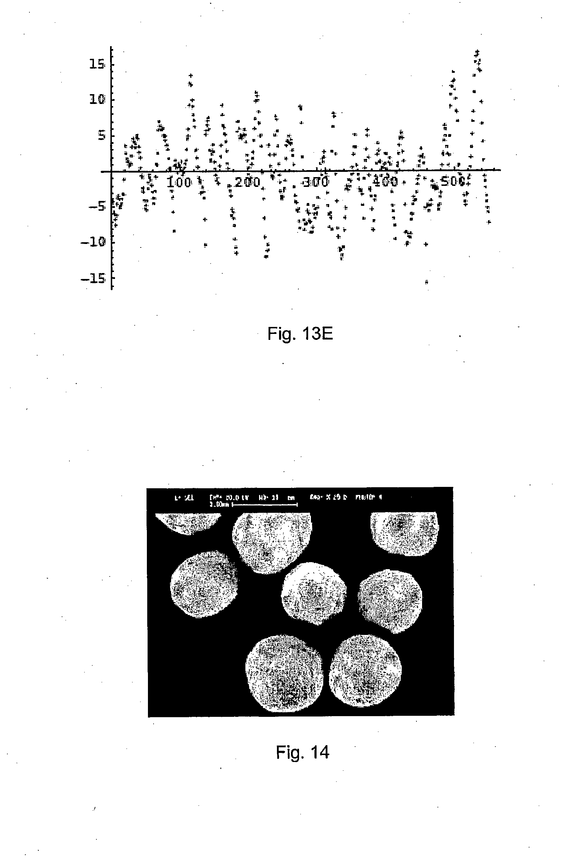

Fig. 13 veranschaulicht durch Abtastung mit einem Laserprofilometer erhaltene Daten, die die Oberfläche eines Pellets der Chargenbezeichnung SFD E 0572 charakterisieren. Dabei ist Fig. 13 A eine grafische Darstellung der Messergebnisse als Oberflächengrafik. In Fig. 13 B sind die Messergebnisse als Höhenliniendiagramm gezeigt. Fig. 13 C zeigt eine Oberflächengrafik auf der Grundlage eines reduzierten Datensatzes. Fig. 13 D zeigt das entsprechende Höhenliniendiagramm. Fig. 13 E zeigt die Abstände der Messpunkte des reduzierten Datensatzes von der nach der Methode der kleinsten Fehlerquadrate ermittelten idealen Oberfläche.

Fig. 14 zeigt eine elektronenmikroskopische Aufnahme (25-fache Vergrößerung) von Pellets der Chargenbezeichnung SFD E 0614, die die erfindungsgemäßen Kriterien für einen Pelletkern mit glatter Oberfläche nicht erfüllen.

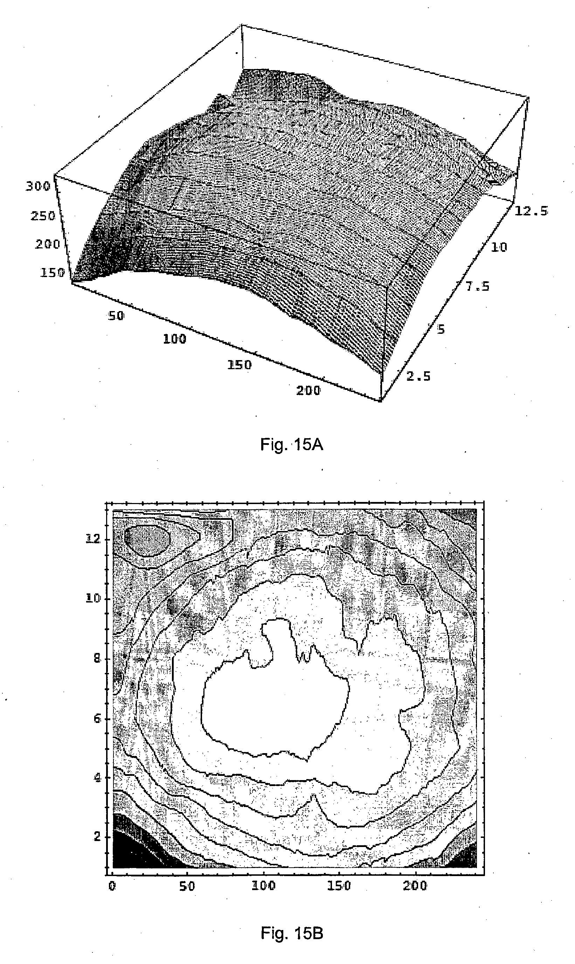

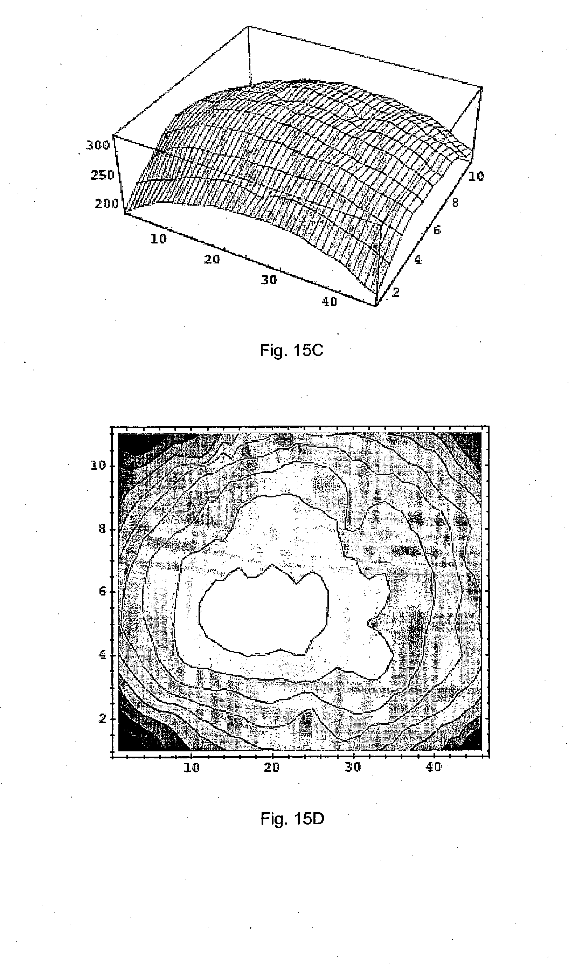

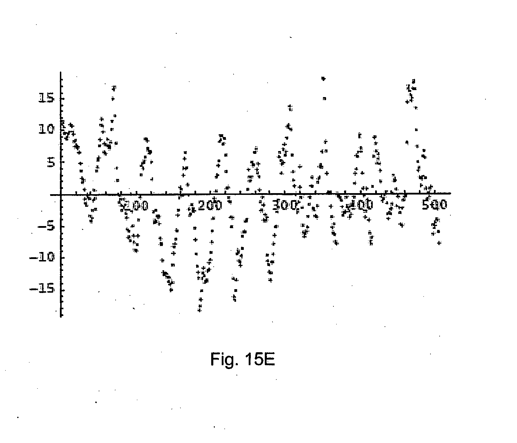

Fig. 15 veranschaulicht durch Abtastung mit einem Laserprofilometer erhaltene Daten, die die Oberfläche eines Pellets der Chargenbezeichnung SFD E 0614 charakterisieren. Dabei ist Fig. 15 A eine grafische Darstellung der Messergebnisse als Oberflächengrafik. In Fig. 15 B sind die Messergebnisse als Höhenliniendiagramm gezeigt. Fig. 15 C zeigt eine Oberflächengrafik auf der Grundlage eines reduzierten Datensatzes. Fig. 15 D zeigt das entsprechende Höhenliniendiagramm. Fig. 15 E zeigt die Abstände der Messpunkte des reduzierten Datensatzes von der nach der Methode der kleinsten Fehlerquadrate ermittelten idealen Oberfläche.

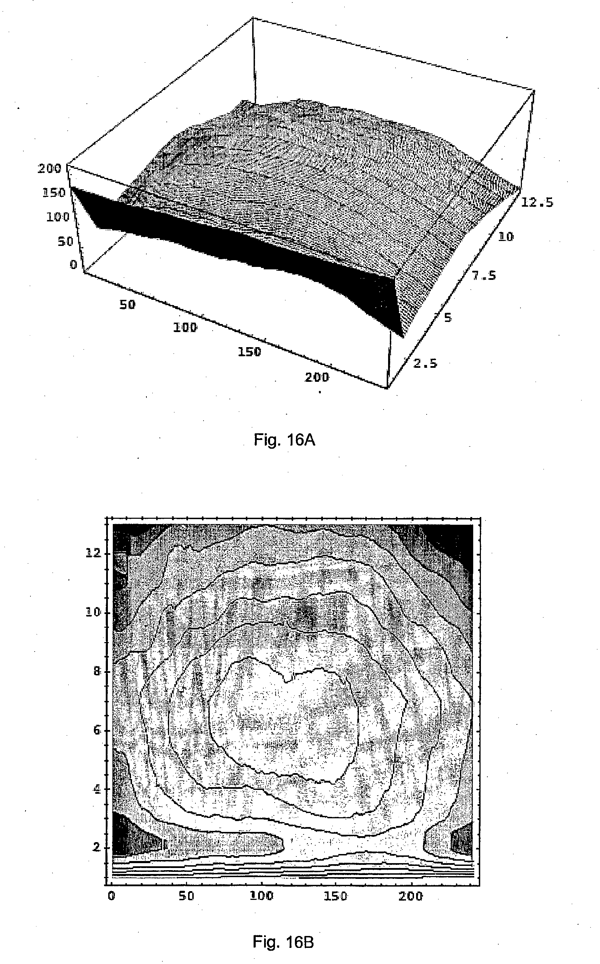

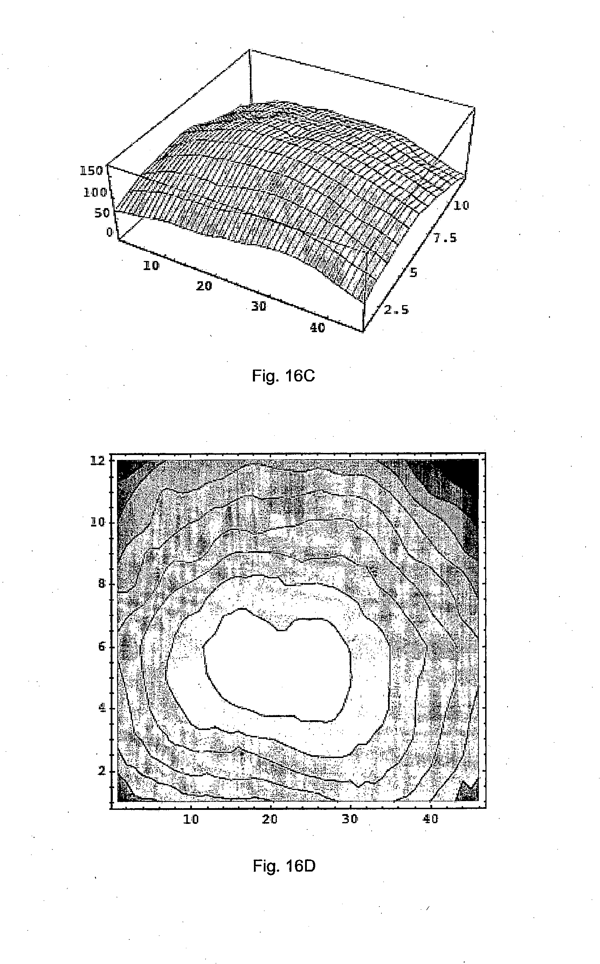

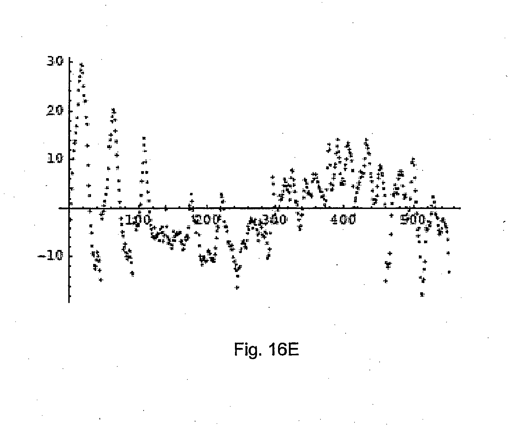

Fig. 16 veranschaulicht durch Abtastung mit einem Laserprofilometer erhaltene Daten, die die Oberfläche eines weiteren Pellets der Chargenbezeichnung SFD E 0614 charakterisieren. Dabei ist Fig. 16 A eine grafische Darstellung der Messergebnisse als Oberflächengrafik. In Fig. 16 B sind die Messergebnisse als Höhenliniendiagramm gezeigt. Fig. 16 C zeigt eine Oberflächengrafik auf der Grundlage eines reduzierten Datensatzes. Fig. 16 D zeigt das entsprechende Höhenliniendiagramm. Fig. 16 E zeigt die Abstände der Messpunkte des reduzierten Datensatzes von der nach der Methode der kleinsten Fehlerquadrate ermittelten idealen Oberfläche.

**Ausführliche Darstellung der Erfindung**

**Definitionen**

Nachstehend werden einige technische Begriffe erläutert, die in der Beschreibung und den Ansprüchen verwendet werden.

Der Ausdruck "Kern" eines pharmazeutischen Pellets soll hier so verstanden werden, dass der gesamte innere Teil des Pellets unterhalb eines Überzugs, der die pH-unabhängige Wirkstofffreisetzung steuert, erfasst ist. Der Kern kann homogen sein oder eine innere Struktur aufweisen. Beispiele umfassen Kerne, bei denen der Wirkstoff homogen in einem Trägermaterial verteilt ist; Kerne, bei denen der Wirkstoff zusammen mit einem oder mehreren Hilfsstoffen in einem Trägermaterial verteilt ist; mit einer Wirkstoffschicht überzogene Startkerne, wobei die Wirkstoffschicht neben dem Wirkstoff noch einen oder mehrere Hilfsstoffe enthalten kann; sowie Kerne der vorstehend genannten Art, die zusätzlich einen oder mehrere Überzüge unter dem die Freisetzung steuernden Überzug aufweisen, beispielsweise Schutzüberzüge oder Trennschichten aus wasserlöslichen Hilfsstoffen, wie wasserlöslichen Filmbildnern.

Als Wirkstoffe kommen erfindungsgemäß beliebige biologisch aktive Substanzen, wie therapeutisch und/oder prophylaktisch wirksame Substanzen, in Betracht, die oral verabreicht werden können.

Der Kern eines pharmazeutischen Pellets weist typischerweise einen Durchmesser im Bereich von 0,2 bis 2 mm, insbesondere von 0,4 bis 1,6 mm und ganz besonders von 0,4 bis 1,4 mm auf.

Ein Pellet oder ein Kern wird als kugelförmig bezeichnet, wenn das Länge-Breite-Verhältnis (d.h. das Verhältnis der Länge (größte Abmessung) des Pellets oder Kerns, dividiert durch die Breite (kleinste Abmessung), die in einem Winkel von 90° in Bezug auf die Länge festgestellt wird) weniger als etwa 1,4 beträgt. Vorzugsweise beträgt das Länge-Breite-Verhältnis eines kugelförmigen Teilchens weniger als etwa 1,3, stärker bevorzugt weniger als etwa 1,2, noch stärker bevorzugt weniger als etwa 1,1 und insbesondere weniger als etwa 1,05.

Erfindungsgemäß haben der kugelförmige wirkstoffhaltige Kern des pharmazeutischen Pellets und typischerweise auch das Pellet selbst eine glatte Oberfläche. Dies bedeutet, dass die Rauheit der Oberfläche eine bestimmte Grenze nicht überschreitet. Die Rauheit wiederum beschreibt die Abweichung einer realen Oberfläche von einer ideal glatten Oberfläche.

Erfindungsgemäß hat es sich dabei als zweckmäßig erwiesen, einen geeigneten Ausschnitt der Oberfläche des Pelletkerns bzw. des Pellets abzutasten, und zwar insbesondere mit optischen Methoden. Aus den Ergebnissen einer solchen Abtastung kann dann ein numerischer Wert für die Rauheit gewonnen werden. Genauer gesagt wird das Oberflächenprofil für einen Ausschnitt der Oberfläche des Pelletkerns bzw. des Pellets untersucht. Der Ausschnitt sollte möglichst so gewählt werden, dass er repräsentativ für die Oberfläche des Teilchens ist. Vermessen wird dann ein Ausschnitt der Oberfläche, der 5 bis 50 %, insbesondere 5 bis 25 %, speziell 5 bis 15 % der gesamten Oberfläche des Pellets entspricht. Für diesen Ausschnitt aus der Oberfläche werden Messpunkte festgelegt, die einem Raster entsprechen. Punkte des Rasters sind typischerweise zwischen 2,5 und 50 $\mu$m, insbesondere zwischen 12,5 und 50 $\mu$m voneinander entfernt. Die Zahl der Messpunkte liegt typischerweise im Bereich von 50 bis 5000.

Die gewonnenen Daten werden sodann mit einer idealen Oberfläche verglichen. Dazu wird angenommen, dass der vermessene Oberflächenbereich sich durch einen Ausschnitt aus einer Kugeloberfläche darstellen lässt. Diese idealisierte Kugeloberfläche kann mathematisch dadurch ermittelt werden, dass der Ausschnitt aus einer Kugeloberfläche berechnet wird, der an die experimentell ermittelte Topografie optimal angepasst ist. Die Funktion, die minimiert wird, ist der mittlere quadratische Abstand der gemessenen Profilpunkte von der Kugeloberfläche. Die freien Parameter sind dabei die Koordinaten des Mittelpunkts der Kugel ($x_{0s}$, $y_{0s}$, $z_{0s}$) sowie der Kugelradius R. Die Rauheit kann dann schließlich über die Abstände $d_i$ der gemessenen Profilpunkte in der idealen Kugeloberfläche bestimmt werden. Dazu wird ein mittlerer quadratischer Wert

$$\sigma_d = \sqrt{\sum_{i=1}^{N} d_i^2} \ / \sqrt{N}$$

ermittelt. In der vorliegenden Anmeldung wird $\sigma_d$ als "mittlere Rauheit" bezeichnet. Die mittlere Rauheit beträgt für einen glatten Pelletkern bzw. ein glattes Pellet typischerweise weniger als 10 $\mu$m und vorzugsweise weniger als 7,5 $\mu$m. Die relative mittlere Rauheit, d.h. die Rauheit dividiert durch den Kugelradius R, beträgt vorzugsweise weniger als 2 %, insbesondere weniger als 1,5 % und ganz besonders bevorzugt weniger als 1,2 %.

Auf der Basis der vorstehend beschriebenen Messungen kann die glatte Beschaffenheit der Oberfläche eines wirkstoffhaltigen Kerns auch durch weitere Parameter beschrieben werden. Ein Parameter ist die maximale Abweichung, d.h. der maximale absolute Abstand eines Profilpunktes von der idealen glatten Oberfläche. Dieser Wert beträgt vorzugsweise nicht mehr als 40 $\mu$m, insbesondere nicht mehr als 30 $\mu$m, noch stärker bevorzugt nicht mehr als 25 $\mu$m und ganz besonders bevorzugt nicht mehr als 20 $\mu$m.

Für ein kugelförmiges Pellet lässt sich auch eine maximale relative Abweichung angeben. Darunter ist die maximale absolute Abweichung, dividiert durch den im Rahmen der Optimierung ermittelten Kugelradius, zu verstehen. Vorzugsweise beträgt die maximale relative Abweichung nicht mehr als 5 %, insbesondere nicht mehr als 3 %.

Ein bevorzugtes Verfahren zur Bestimmung der Rauheit eines Pellets wird an späterer Stelle beschrieben.

Eine pH-unabhängige Wirkstofffreisetzung bedeutet, dass die Wirkstofffreisetzung nicht signifikant variiert, wenn erfindungsgemäße Pellets Medien mit pH-Werten ausgesetzt werden, wie sie in verschiedenen Abschnitten des Magen-Darm-Trakts angetroffen werden. Diese pH-Werte liegen im Bereich von 1,0 bis 8,0.

Methoden zur Bestimmung der Wirkstofffreisetzung sind in der USP (amerikanisches Arzneibuch) und in der PH. EUR. definiert. Auf diese Methoden wird hier Bezug genommen. Insbesondere wird eine Paddle-Apparatur verwendet. Die Rührgeschwindigkeit beträgt 50 Umdrehungen pro Minute. Die Temperatur des Testmediums beträgt 37°C. Als Testmedium wird ein Phosphatpuffer mit einem pH-Wert von 6,8 verwendet.

Gemäß einem Aspekt der Erfindung erfolgt die Wirkstofffreisetzung mit einem bestimmten Profil. Das Profil weist eine Lag-Phase auf, wobei während der Lag-Phase ein Anteil von 5 Gew.-% oder weniger des Wirkstoffs freigesetzt wird. Die Lag-Phase dauert 60 bis 840 Minuten, vorzugsweise 60 Minuten bis 540 Minuten.

Gemäß einem Aspekt der Erfindung werden nach einer Lag-Phase innerhalb von 60 Minuten, vorzugsweise innerhalb von 30 Minuten mindestens 80 Gew.-% des noch verbliebenen Wirkstoffs freigesetzt. Gemäß einem weiteren Aspekt der Erfindung beträgt die Freisetzung des Wirkstoffs nach einer Lag-Phase zwischen 3 und 25 Gew.-% pro Stunde, vorzugsweise zwischen 3 und 15 Gew.-% pro Stunde und insbesondere zwischen 3 und 6,5 Gew.-% pro Stunde.

**Bevorzugte Ausführungsformen - Pellets**

Erfindungsgemäße pharmazeutische Pellets weisen einen Kern auf, der einen oder mehrere Wirkstoffe enthält. Bevorzugt sind Wirkstoffe mit hoher Wasserlöslichkeit. Weiterhin bevorzugt sind wasserlösliche Salze von schwer wasserlöslichen Wirkstoffen. Ein Beispiel hierfür ist ADD 006 mit einer Wasserlöslichkeit von ca. 210 mg/ml bei 25°C.

Der Kern enthält weiterhin typischerweise ein oder mehrere Bindemittel. Bevorzugt sind wasserlösliche Bindemittel. Dazu gehören Calciumcarboxymethylcellulose, Polymere auf Acrylsäurebasis (Carbopol), Gelatine, Hydroxyethylcellulose, Hydroxypropylcellulose, Hydroxypropylmethylcellulose, Polyethylenglycol (Macrogol), Methylcellulose, Natriumcarboxymethylcellulose, Natriumcarboxymethylstärke, Polyoxypropylen-Polyoxyethylen-Blockpolymere (Poloxamer), Polyvinylalkohol, Polyvinylpyrrolidon (Povidon) und Stärke. Bevorzugte Bindemittel umfassen Gelatine, Natriumcarboxymethylcellulose und Polyvinylpyrrolidon (Povidon). Ganz besonders bevorzugt ist Polyvinylpyrrolidon. Polyvinylpyrrolidon (Povidon) ist in geeigneter Form im Handel erhältlich, beispielsweise als Kollidon 30.

Der Kern kann außerdem Trägerstoffe oder Füllstoffe enthalten. Geeignet sind beispielsweise Kohlenhydrate. Als Beispiele können wasserlösliche Kohlenhydrate genannt werden, wie Dextran, Dextrin, Dextrose (Glucose), Fructose, Lactose, Maltodextrin, Mannitol, Saccharose, Sorbitol und Xylitol. Ein anderes Beispiel für einen Trägerstoff oder Füllstoff ist mikrokristalline Cellulose.

Der Kern kann weitere optionale Bestandteile enthalten. Es können sowohl wasserlösliche als auch wasserunlösliche Hilfsstoffe zusätzlich enthalten sein. Dazu gehören Konservierungsmittel, physikalische Stabilisatoren und chemische Stabilisatoren, wie saure, basische oder puffernde Komponenten. Weiterhin können Netzmittel und Lösungsvermittler enthalten sein. Die Kerne können ferner osmotisch aktive Substanzen enthalten. Dies ist insbesondere bei Wirkstoffen bevorzugt, die nur wenig wasserlöslich sind. Hier kann eine osmotisch aktive Substanz als Schleppmittel dienen. Zu den optionalen Bestandteilen gehören auch Antiklebmittel.

Der Pelletkern kann mit einer Schutzschicht überzogen sein. Bevorzugte Materialien sind wasserlösliche Polymere, insbesondere Hydroxypropylmethylcellulose.

Erfindungsgemäß sollen die Pelletkerne, die mit dem die Wirkstofffreisetzung steuernden Überzug versehen werden, möglichst rund und möglichst glatt sein. Um die Qualität von Pelletkernen bzw. Pellets überprüfen zu können, wird daher eine Obergrenze für die Rauheit festgelegt. Um die Rauheit eines Pellets zu bestimmen, wird das Oberflächenprofil eines geeignet gewählten Ausschnitts aufgezeichnet, vorzugsweise berührungsfrei mit optischen Methoden, beispielsweise unter Verwendung eines autofokussierenden Laserprofilometers, wie eines Laserprofilometers UBM. Dessen Funktionsweise kann wie folgt beschrieben werden:

Ein Laserstrahl wird über eine Optik auf die Oberfläche der zu vermessenden Struktur (Ausschnitt aus der Oberfläche eines Pelletkerns oder Pellets) abgebildet. Der Sensorkopf besteht aus einem Linsensystem und zwei zu dessen optischer Achse symmetrisch angeordneten Fotodetektoren. Auf der Höhe des Fokuspunktes des Linsensystems ist eine Blende angebracht, die das Abbild des Laserstrahls einseitig beschneidet. Befindet sich die Probe im Fokus des Sensors, so wird der an der Probe reflektierte Laserstrahl im Bereich der Blende fokussiert und nicht beschnitten. In diesem Fall messen beide Detektoren die gleiche Intensität. Befindet sich die Probe außerhalb des Fokusbereichs, so wird kein scharfes Bild des Laserstrahls erzeugt. Vielmehr liegt im Bereich der Blende eine breitere Intensitätsverteilung vor, die durch diese beschnitten wird. Als Folge messen die Detektoren unterschiedliche Intensitäten. Zur Messung der Höhendifferenz $\Delta h$ wird der Sensorkopf so lange vertikal verfahren, bis sich die Probe wieder im Fokus befindet und beide Detektoren die gleiche Intensität messen. Die vertikale Verschiebung des Sensors, die über magnetische Stellelemente erfolgt, gibt dann den Höhenunterschied wieder. Während der Sensorkopf kontinuierlich die Höhe der Probe vermisst, wird diese über motorisierte x-y-Tische unter dem Sensorkopf hinwegbewegt. Die laterale Auflösung des Profilometers liegt dabei bei 0,5 $\mu$m, die im Wesentlichen durch den Strahldurchmesser bestimmt wird. Die vertikale Genauigkeit wird vom Hersteller mit 100 nm angegeben.

Der zu vermessende Ausschnitt aus der Oberfläche wird so festgelegt, dass typische Profilstrukturen erfasst werden. Beispielsweise kann bei einem kugelförmigen Pellet mit einem Radius zwischen 400 und 600 $\mu$m ein Oberflächenbereich von 600 $\mu$m x 600 $\mu$m abgetastet werden. Die Abtastung erfolgt in dreizehn parallelen Linien-Scans in Abständen von 50 $\mu$m. Bei den einzelnen Linien-Scans erfolgt die Abtastung in Intervallen von 2,5 $\mu$m, was zu einer Gesamtzahl von 241 Punkten pro Linien-Scan führt.

Die mathematische Auswertung der Daten ist typischerweise mit einer Datenreduktion verbunden. Während des Beginns der Zeilenabtastung muss das optische Instrument zunächst eine Fokussierung auf die Oberfläche erreichen. Die ersten Messungen sind aus diesem Grund mit Unsicherheiten behaftet. Daher werden die ersten fünfzehn Messpunkte jeder Zeilenabtastung von der Analyse ausgeschlossen. Da gelegentlich bei den ersten Zeilenabtastungen keine Konvergenz der Fokussierprozedur erreicht wird, was seine Ursache darin haben kann, dass Startpunkt oder Endpunkt der Zeilenabtastung außerhalb des Perimeters des kugelförmigen Teilchens liegen, werden solche Zeilenabtastungen von der Analyse ausgeschlossen. Ähnliche Fokussierprobleme treten gelegentlich bei den letzten Zeilenabtastungen auf, die dann ebenfalls von der Analyse ausgeschlossen werden.

Die Daten der Zeilenabtastung werden um einen Faktor 5 ausgedünnt, was zu einer Verringerung der 241 - 15 Messpunkte pro Zeile auf typischerweise 45 Messpunkte pro Zeile führt. Experimentell hat sich gezeigt, dass dies zu keinem signifikanten Verlust einer Oberflächenprofilstruktur führt, da typische Profilstrukturen sich über 50 bis 100 $\mu$m erstrecken.

Wie bereits angegeben, wird dann ein Abschnitt aus einer idealen Kugeloberfläche nach der Methode der kleinsten Fehlerquadrate an den reduzierten Satz der Datenpunkte angepasst. Abweichungen von der idealen Oberfläche werden als Maß für die Rauheit herangezogen. Dazu kann eine mittlere Rauheit $\sigma_d$ definiert werden.

Dieser Texturparameter entspricht der üblichen Definition der weit verbreitet für planare texturierte Oberflächen herangezogenen mittleren quadratischen Rauheit $R_{RMS}$. Zusätzlich wird noch eine relative mittlere quadratische Rauheit definiert, die sich aus der vorstehend definierten mittleren Rauheit $\sigma_d$ und dem Kugelradius R gemäß $\sigma_d/R$ berechnen lässt. Die relative mittlere quadratische Rauheit wird in der vorliegenden Anmeldung auch in Prozent angegeben ($\sigma_d/R$ x 100%).

Pelletkerne mit glatter Oberfläche, wie sie vorstehend beschrieben wurden, sind erfindungsgemäß mit einem Überzug versehen, der pH-unabhängig die Wirkstofffreisetzung steuert.

Die Überzüge zur pH-unabhängigen Wirkstofffreisetzung enthalten vorzugsweise ein Polymer, das unter Ammonium-methacrylat-copolymer, Celluloseacetatbutyrat, Celluloseacetat, Celluloseacetatpropionat, Ethylcellulose, Poly(ethylacrylat-methylacrylat) und Polyvinylacetat ausgewählt ist. Besonders bevorzugt ist Polyvinylacetat. Bei Verwendung derartiger Filmbildner kann die Wirkstofffreisetzung aus den Pellets durch die Schichtdicke des aufgebrachten Films und durch die Auswahl geeigneter zusätzlicher Hilfsstoffe (insbesondere Porenbildner) modifiziert werden.

Entsprechend kann der Überzug zur Steuerung der pH-unabhängigen Wirkstofffreisetzung Porenbilder enthalten, insbesondere wasserlösliche Polymere oder sonstige wasserlösliche Verbindungen.

Der Überzug zur Steuerung der pH-unabhängigen Wirkstofffreisetzung kann auch Weichmacher enthalten. Dazu gehören Acetyltributylcitrat, Triacetin, acetyliertes Monoglycerid, Rapsöl, Olivenöl, Sesamöl, Acetyltriethylcitrat, Glycerinsorbitol, Diethyloxalat, Diethylmalat, Diethylfumarat, Dibutylsucchinat, Diethylmalonat, Dioctylstalat, Dibutylsebacat, Diethylcitrat, Tributylcitrat, Glycerol, Tributyrat, Polyethylenglykol, Propylenglykol und Gemische davon.

In dem Überzug kann auch ein Trennmittel enthalten sein. Ein Beispiel dafür ist Talkum.

Die erfindungsgemäßen Pellets können auch einen äußeren Schutzüberzug aufweisen. Bevorzugte Materialien dafür sind wasserlösliche Polymere, insbesondere Hydroxypropylmethylcellulose.

Unter Verwendung der erfindungsgemäßen Pellets können multipartikuläre Darreichungsformen bereitgestellt werden. Beispielsweise können die Pellets in Kapseln gefüllt werden. Aus den Pellets können aber auch Tabletten hergestellt werden.

**Bevorzugte Ausführungsformen - Herstellungsverfahren**

Erfindungsgemäß werden auch Herstellungsverfahren bereitgestellt. Dazu gehören Verfahren zur Herstellung von Pelletkernen sowie Verfahren zum Überziehen von Pelletkernen, wobei die Erfindung aber nicht auf eine bestimmte Herstellungsweise beschränkt ist.

Ein Verfahren zur Herstellung von Pelletkernen ist die Wirbelschichtagglomeration. Die Granulation kann ohne Startkerne durchgeführt werden. Es können dadurch Pelletkerne mit einem vergleichsweise sehr hohen Wirkstoffanteil hergestellt werden. Beispielsweise können Pelletkerne hergestellt werden, die 80 Gew.-Teile oder mehr, vorzugsweise 90 Gew.-Teile oder mehr, an Wirkstoff sowie außerdem ein Bindemittel enthalten. Mit Hilfe eines Sichters, mit dem kontinuierlich Pelletkerne aus dem Prozess entnommen werden, lässt sich eine sehr enge Korngrößenverteilung erzielen. Z.B. kann ein Pelletkernprodukt erhalten werden, bei dem 95 Gew.-% oder mehr, vorzugsweise 99 Gew.-% oder mehr, der Pelletkerne eine Größe zwischen 100 und 300 $\mu$m oder auch zwischen 200 und 300 $\mu$m aufweisen.

Zur Durchführung des Verfahrens wird eine flüssige, vorzugsweise wässrige Lösung oder Dispersion mit einem Wirkstoff und ggf. einem oder mehreren Hilfsstoffen, wie einem Bindemittel, zu Beginn des Prozesses von unten in eine leere Wirbelschichtanlage eingesprüht. Durch die Sprühgranulation bilden sich Startkerne für die Pelletierung. Durch Anlagerung weiteren Materials erreichen die Kerne schließlich eine solche Größe, dass sie über einen Sichter aus der Anlage austreten.

Geeignete Vorrichtungen, um eine Wirbelschichtagglomeration durchzuführen, sind in EP 163 836 B1, EP 332 031 B1 sowie in EP 332 929 B1 beschrieben.

Ein weiteres Verfahren zur Herstellung von Pelletkernen umfasst die Bereitstellung von Startkernen und das anschließende Aufbringen einer Wirkstoffschicht (Layering). Ein Wirkstoff, ggf. zusammen mit weiteren Inhaltsstoffen, wird dabei auf Kerne aufgebracht. Dies kann in einer Wirbelschichtauflage erfolgen, wobei ein pulverförmiges Material und eine Flüssigkeit, die eine Bindung der Pulverbestandteile an die Kerne erlaubt, zugeführt werden. Bei dem Pulver kann es sich um einen Wirkstoff oder ein Gemisch aus einem Wirkstoff und einem oder mehreren Hilfsstoffen handeln. Bei der Flüssigkeit kann es sich um Wasser oder ein organisches Lösungsmittel handeln; die Flüssigkeit kann auch eine Lösung oder Dispersion sein.

Bevorzugt ist außerdem ein Verfahren zur Herstellung von wirkstoffhaltigen Pelletkernen, bei dem Starterkerne, beispielsweise in einer Wirbelschichtapparatur, mit einer Lösung oder Dispersion, die einen Wirkstoff und ggf. einen oder mehrere Hilfsstoffe enthält, überzogen werden.

Die vorstehend beschriebene Herstellung von Pelletkernen durch Aufbringen einer Wirkstoffschicht auf Startkerne erfolgt vorzugsweise in einer Wirbelschichtapparatur mit Wurster-Einsatz (nach dem Wurster-Verfahren).

Ein weiteres Verfahren zur Herstellung von Pelletkernen umfasst in einer ersten Stufe die Bereitstellung eines Ausgangsmaterialpulvers. Das Ausgangsmaterialpulver umfasst einen Träger. Das Ausgangsmaterialpulver wird vorzugsweise einer Behandlung, beispielsweise in einem Schermischer, unterzogen, um Aggregate zu zerstören. Bei dem Ausgangsmaterialpulver kann es sich auch um ein Gemisch aus einem Träger und einem oder mehreren weiteren Inhaltsstoffen der herzustellenden Pelletkerne, beispielsweise Bindemittel, handeln. In diesem Fall beinhaltet die Bereitstellung des Ausgangsmaterialpulvers typischerweise das Mischen der Komponenten.

Vorzugsweise wird ein Ausgangsmaterialpulver verwendet, dessen Korngröße begrenzt ist. Insbesondere umfasst das Ausgangsmaterialpulver einen Träger, dessen Korngröße begrenzt ist. Weiterhin ist eine enge Korngrößenverteilung für das Ausgangsmaterialpulver und insbesondere für den Träger bevorzugt.

Beispielsweise hat es sich bei der Herstellung von Pelletkernen mit einer Größe von 300 bis 500 $\mu$m bewährt, ein Ausgangsmaterialpulver, insbesondere einen Träger, zu verwenden, bei dem der Siebrückstand bei einer Siebmaschenweite von 160 $\mu$m weniger als 10 Gew.-% und insbesondere weniger als 5 Gew.-% beträgt. Weiterhin bevorzugt sind Ausgangsmaterialpulver, insbesondere Pulver eines Trägers, bei denen der Siebrückstand bei einer Siebmaschenweite von 40 $\mu$m im Bereich von 50 bis 80 Gew.-% liegt. Besonders bevorzugt liegt der Siebrückstand bei einer Maschenweite von 160 $\mu$m im Bereich von 1 bis 5 Gew.-% und bei einer Maschenweite von 40 $\mu$m im Bereich von 50 bis 70 Gew.-%.

Das Ausgangsmaterialpulver kann vor der Stufe des Pelletierens befeuchtet werden. Hierzu wird es mit einem pharmazeutisch annehmbaren Verdünnungsmittel versetzt. Dabei kann es sich um das gleiche Verdünnungsmittel wie das, das in der nachfolgenden Pelletierstufe eingesetzt wird, oder um ein anders zusammengesetztes Verdünnungsmittel handeln. Bei dem Verdünnungsmittel kann es sich um eine organische Flüssigkeit handeln. Vorzugsweise handelt es sich um Wasser oder eine wässrige Lösung oder Dispersion. In der Flüssigkeit können als Bestandteile ein Bindemittel und/oder ein Wirkstoff und/oder weitere Kerninhaltsstoffe vorliegen. Die Menge des pharmazeutisch annehmbaren Verdünnungsmittels wird vorzugsweise so bemessen, dass ein benetztes Ausgangsmaterialpulver erzielt wird, wobei die zugesetzte Flüssigkeitsmenge unterhalb der Menge liegt, die zur Ausbildung von Granulatstrukturen erforderlich ist. Vorzugsweise wird für eine gleichmäßige Befeuchtung des Ausgangsmaterialpulvers gesorgt. Dies kann unter Verwendung eines geeigneten Mischers, wie eines Schermischers, erfolgen.

Werden mehrere pulverförmige Komponenten eingesetzt, können Mischen und Vorbefeuchten in einem Schritt, beispielsweise in einem Schnellmischer, erfolgen.

In der Pelletierstufe werden aus dem gegebenenfalls vorbefeuchteten Ausgangsmaterial unter Zugabe eines pharmazeutisch annehmbaren flüssigen Verdünnungsmittels Pellets geformt. Für die Beschaffenheit des Verdünnungsmittels gelten die gleichen Kriterien wie für das zur Vorbefeuchtung verwendete Verdünnungsmittel.

Das Verdünnungsmittel kann einen Wirkstoff enthalten.

Es ist auch möglich, pulverförmige Bestandteile, beispielsweise pulverförmigen Wirkstoff, während der Pelletierung zuzuführen, wenn eine homogene Durchmischung durch die Prozessführung gegeben ist. Da die erfindungsgemäßen Pellets einen Wirkstoff enthalten, muss dieser entweder in dem Ausgangsmaterialpulver enthalten sein oder während der Pelletierung als Bestandteil des Verdünnungsmittels oder in Pulverform zugeführt werden. Kombinationen dieser Maßnahmen sind auch möglich.

Gemäß einer bevorzugten Ausführungsform umfasst das Verfahren folgende Stufen:

(a) Bereitstellung eines Ausgangsmaterialpulvers, das einen Träger umfasst;

(b) Einführen des Ausgangsmaterialpulvers, das wahlweise mit einem pharmazeutisch geeigneten flüssigen Verdünnungsmittel benetzt ist, in eine Vorrichtung, die aufweist:

eine Rotorkammer mit einer sich axial erstreckenden zylindrischen Wand,

eine Einrichtung, um Luft durch die Rotorkammer vom Boden aus zu leiten,

eine Sprüheinrichtung für die Zufuhr von Flüssigkeit in die Kammer,

eine oder mehrere Einlassöffnungen zur Einführung des Pulvergemisches,

einen Rotor, der sich um eine vertikale Rotorachse dreht, wobei der Rotor in der Rotorkammer angeordnet ist, eine zentrale horizontale Oberfläche und in mindestens dem äußeren Drittel des Rotors die Form einer konischen Mantelfläche mit einer nach außen und oben gerichteten Neigung zwischen 10° und 80° aufweist, wobei die konische Mantelfläche eine kreisförmige obere Kante aufweist, die in einer Ebene liegt, die senkrecht zu der Rotorachse ist,

eine Mehrzahl von Leitschaufeln jeweils mit einem äußeren Ende, das statisch an der zylindrischen Wand der Rotorkammer oberhalb der Ebene befestigt ist, die von der oberen Kante der konischen Mantelfläche des Rotors gebildet wird, und einem inneren Ende, das sich in die Rotorkammer erstreckt und tangential zu der zylindrischen Wand der Rotorkammer angeordnet ist und im Querschnitt zur Rotorachse im Wesentlichen die Form eines Kreisbogens oder einer Spirale aufweist,

(c) Rotation des Rotors, so dass das Produkt, das durch kinetische Energie für einen ausreichenden Zeitraum im Kreis geführt wird, sich von dem Rotor zu der inneren Oberfläche der Leitschaufeln bewegt, bevor es zurück auf den Rotor fällt, während optional Luft zugeführt und/oder eine pharmazeutisch annehmbare Flüssigkeit in die Rotorkammer gesprüht wird, so dass feste Pellets mit einem gewünschten Durchmesser gebildet werden;

Eine geeignete Vorrichtung zur Ausführung des ersten Verfahrens zur Herstellung von Pelletkernen ist in DE 197 50 042 A1 beschrieben.

Während der Pelletbildung wird ein pharmazeutisch annehmbares Verdünnungsmittel zugeführt, wie dies vorstehend beschrieben wurde. Die Menge wird insbesondere in Abhängigkeit von den Komponenten des Ausgangsmaterials, der gewünschten Pelletgröße sowie den sonstigen Betriebsparametern, beispielsweise der Menge an zugeführter Luft, gewählt.

Die schließlich erhaltenen Pelletkerne werden getrocknet.

Vorzugsweise wird überprüft, ob die hergestellten Pelletkerne den Anforderungen der Erfindung genügen. Insbesondere wird überprüft, ob ein erfindungsgemäßes Pelletkernprodukt vorliegt. Dies bedeutet, dass vorzugsweise die Pellets des erhaltenen Pelletkernprodukts, das aus einem Kollektiv oder einer Mehrzahl von Pelletkernen besteht, überwiegend kugelförmig sind und glatte Oberflächen aufweisen. Vorzugsweise erfüllen mindestens 90 % der Pelletkerne eines Pelletkernprodukts die erfindungsgemäßen Anforderungen hinsichtlich kugelförmiger Gestalt und glatter Oberfläche.

Die erhaltenen Pellets können als Pelletprodukt, das eine Mehrzahl von Pellets umfasst, Verwendung finden. Ein Pelletprodukt umfasst ein Kollektiv von Pellets, typischerweise 50 oder mehr, vorzugsweise 100 oder mehr Pellets. Ein erfindungsgemäßes Pelletprodukt umfasst überwiegend Pellets, die die erfindungsgemäßen Kriterien erfüllen. Vorzugsweise weisen mindestens 90 %, insbesondere mindestens 95 % und ganz besonders bevorzugt mindestens 98 % der Pellets ein Länge-Breite-Verhältnis von weniger als etwa 1,4, vorzugsweise weniger als etwa 1,3, stärker bevorzugt weniger als etwa 1,2, noch stärker bevorzugt weniger als etwa 1,1 und insbesondere weniger als etwa 1,05 auf. Die Pellets, die die bevorzugten Länge-Breite-Verhältnisse aufweisen, weisen vorzugsweise auch die weiteren Anforderungen an erfindungsgemäße Pellets auf, speziell auch die Anforderungen, die in den Ansprüchen und in der Beschreibung niedergelegt sind.

Die nach einem der vorstehenden Verfahren erhaltenen Pelletkerne können ggf. nach an sich bekannten Verfahren mit einem oder mehreren Überzügen versehen werden. Dazu gehören Überzüge aus wasserlöslichen Filmbildnern.

Die Pelletkerne - mit oder ohne Überzug, wie einem Überzug aus einem wasserlöslichen Filmbildner, werden mit einem Polymer zur pH-unabhängigen Wirkstofffreisetzung überzogen.

Erfindungsgemäß können außerdem Tabletten hergestellt werden, indem pharmazeutische Pellets, ggf. zusammen mit Hilfsstoffen, verpresst werden. Dabei ist es erfindungsgemäß möglich, Tabletten zu erhalten, wobei die Freisetzungskurve des Wirkstoffs im Vergleich zur Freisetzungskurve aus den Pellets parallel verschoben ist. Mit anderen Worten ist die Lag-Phase, die bei der Freisetzung aus den Tabletten auftritt, gegenüber der Lag-Phase, die bei der Freisetzung aus den Pellets auftritt, verkürzt, während die Steilheit der Freisetzungskurve im Wesentlichen unverändert bleibt. Das Freisetzungsverhalten kann gemessen werden, wie es bereits vorstehend beschrieben wurde.

Die nachstehenden Herstellungsbeispiele, Beispiele und Testbeispiele erläutern die Erfindung. Als Wirkstoff kommt dabei ADD 006 zum Einsatz, dessen Wasserlöslichkeit bei 25°C ca. 210 mg/ml beträgt.

Herstellungsbeispiel 1

Das Beispiel betrifft die Herstellung von ADD 006 enthaltenden Pellets.

Zur Herstellung von Pelletkernen mit dem Wirkstoff ADD 006 werden folgende Ausgangsmaterialien verwendet:

| | |
|---|---|
| Sugar spheres (Starterkerne) | 300 g |
| Syloid 244 FP (Präzipitiertes Siliciumdioxid) | 45 g |
| ADD 006 | 1200 g |
| Entmineralisiertes Wasser | 2757 g |

Ein Anteil des entmineralisierten Wassers (2457 g) wird auf ungefähr 60°C erwärmt. Das ADD 006 wird in dem erwärmten Wasser gelöst. Das Siliciumdioxid (Syloid 244 FP) wird in einem weiteren Anteil des entmineralisierten Wassers (300 g) mit einem Homogenisator zehn Minuten suspendiert. Anschließend wird die Siliciumdioxid-Dispersion in die Wirkstofflösung gegossen. Das resultierende Gemisch (Überzugsdispersion) wird unter Rühren bei einer Temperatur von ungefähr 50°C gehalten.

Zum Überziehen werden die Starterkerne in eine Wirbelschicht-Beschichtungsvorrichtung (Glatt GPCG I) gegeben. Anschließend wird die Überzugsdispersion mit einer Sprührate von zu Beginn 6 g/min aufgesprüht. Die Sprührate wird anschließend auf 8 bis 10 g/min erhöht. Die Einlasslufttemperatur beträgt während des Überziehens ca. 55°C.

Nachdem die Überzugsdispersion aufgesprüht ist, werden die erhaltenen Kerne in der Apparatur für zehn Minuten bei einer Einlasslufttemperatur von ungefähr 60°C getrocknet. Anschließend werden die Kerne entnommen und über Nacht bei 45°C nachgetrocknet.

Auf die vorstehend beschriebene Weise können ADD 006-Kerne mit einem Wirkstoffgehalt von 77,67 Gew.-% erhalten werden.

Die erhaltenen wirkstoffhaltigen Kerne können mit einem Überzug versehen werden, der pH-unabhängig die Wirkstofffreisetzung steuert. Ein geeignetes Überzugsmaterial ist Polyvinylacetat. Der Beispielansatz geht von folgenden Ausgangsmaterialien aus:

| | |
|---|---|
| ADD 006-Kerne (wie vorstehend beschrieben) | 300,00 g |
| Kollicoat SR 30 D (Polyvinylacetat-Dispersion) | 277,78 g (Feststoffgehalt: 83,33 g) |
| Avicel PH 105 (mikrokristalline Cellulose) | 5,00 g |
| Triethylcitrat | 8,33 g |
| Talkum | 8,33 g |
| Entmineralisiertes Wasser | 280,00 g |

Zur Herstellung der Überzugsdispersion wird zunächst die mikrokristalline Cellulose in dem entmineralisierten Wasser unter Verwendung eines Homogenisators dispergiert und für fünf Minuten gerührt. Sodann werden Triethylcitrat und Talkum zu der Dispersion gegeben und die Dispersion wird erneut für zehn Minuten homogenisiert. Die Polyvinylacetat-Dispersion wird gesiebt und unter Rühren langsam mit der vorstehend erhaltenen Hilfsstoffdispersion versetzt. Die erhaltene Überzugsdispersion wird für eine Stunde gerührt, bevor mit der Befilmung der Kerne begonnen wird.

Das Aufbringen des Überzugs erfolgt in einer Wirbelschichtbeschichtungsvorrichtung (Glatt GPCG I). Die wirkstoffhaltigen Kerne werden auf ungefähr 30°C erwärmt. Anschließend wird die Überzugsdispersion mit einer Sprührate von ungefähr 7 bis 8 g/min aufgesprüht. Die Einlasslufttemperatur beträgt dabei ungefähr 35°C.

Unter den gleichen Bedingungen wie vorstehend wird anschließend zunächst noch eine erste äußere Pelletphase aufgebracht. Dabei werden folgende Hilfsstoffe in folgenden Mengen verwendet:

| | |
|---|---|
| Syloid 244 FP (gefälltes Siliciumdioxid) | 7,03 g |
| Pharmacoat 603 (Hydroxypropylmethylcellulose) | 1,07 g |
| Entmineralisiertes Wasser | 50,81 g |

Hydroxypropylmethylcellulose wird in dem Wasser gelöst, und anschließend wird das Siliciumdioxid portionsweise unter Rühren zugegeben. Nach dem Aufbringen dieser Dispersion auf die überzogenen Pellets werden die Pellets aus der Wirbelschichtapparatur entnommen. Sie werden dann noch mit folgenden Bestandteilen als zweiter äußerer Pelletphase versetzt:

| | |
|---|---|
| Syloid 244 FP (gefälltes Siliciumdioxid) | 4,13 g |

(fortgesetzt)

| Talkum | 4,13 g |
|---|---|

Die mit diesen Hilfsstoffen versetzten Pellets werden für fünf Minuten in einem Diffusionsmischer (Turbula T2C) gemischt. Das erhaltene Produkt wird über Nacht in einem belüfteten Ofen bei 60°C nachgetrocknet.

**Herstellungsbeispiel 2**

Das Beispiel betrifft die Herstellung von ADD 006 enthaltenden Pellets sowie die Weiterverarbeitung der Pellets zu Tabletten.

Die Herstellung von Pelletkernen mit dem Wirkstoff ADD 006 erfolgt in der gleichen Weise wie in Herstellungsbeispiel 1. Für ein Präparat mit einer Dosisstärke von 200 mg ADD 006 werden Ausgangsmaterialien in folgenden Mengenanteilen verwendet:

| Sugar spheres NF (Starterkerne) | 50,000 mg |
|---|---|
| ADD 006 | 200,000 mg |
| Kolloidales Siliciumdioxid | 7,500 mg |
| Zwischensumme | 257,500 mg |

Die wirkstoffhaltigen Pelletkerne werden mit einem Überzug zur kontrollierten Freisetzung versehen. So werden zwei verschiedene Formulierungen untersucht. Die Mengenanteile in der ersten Formulierung, bezogen auf 257,500 mg wirkstoffhaltige Kerne, lauten wie folgt:

| Polyvinylacetat-Dispersion, Feststoffe | 75,100 mg |
|---|---|
| Talkum (10 %) | 7,500 mg |
| Triethylcitrat (10%) | 7,500 mg |
| Zwischensumme | 90,100 mg |

In der zweiten Formulierung für den Überzug zur kontrollierten Freisetzung wird eine geringere Menge an Weichmacher (Triethylcitrat) eingesetzt. Die Mengenanteile der Formulierung, bezogen auf 257,500 mg wirkstoffhaltige Pelletkerne, lauten wie folgt:

| Polyvinylacetat-Dispersion, Feststoffe | 76,700 mg |
|---|---|
| Talkum (10 %) | 7,700 mg |
| Triethylcitrat (7,5 %) | 5,800 mg |
| Zwischensumme | 90,100 mg |

Auf der Basis der Formulierungen werden überzogene Pellets hergestellt, wie es in Herstellungsbeispiel 1 beschrieben wurde. Auf die Pellets wird anschließend ein Schutzüberzug (2 %) aufgebracht. Die Mengenanteile der Komponenten dieses Schutzüberzugs sind wie folgt:

| Hydroxypropylmethylcellulose | 3,500 mg |
|---|---|
| Kolloidales Siliciumdioxid | 3,500 mg |
| Zwischensumme | 7,000 mg |

Die erhaltenen Pellets werden anschließend verwendet, um Tabletten herzustellen. Die Tablettierphase enthält folgende Mengenanteile an Hilfsstoffen:

| Mikrokristalline Cellulose | 105,9 mg |
|---|---|

(fortgesetzt)

| | |
|---|---|
| Lactose | 337,3 mg |
| Maisstärke | 53,0 mg |
| Crospovidon | 26,5 mg |
| Kolloidales Siliciumdioxid | 1,6 mg |
| Magnesiumstearat | 5,3 mg |
| Zwischensumme | 529,6 mg |

Die mit dem die Wirkstofffreisetzung steuernden Überzug und dem Schutzüberzug versehenen Pellets werden mit den genannten Hilfsstoffen gemischt und zu Tabletten verpresst. Es werden Tabletten mit einem Gewicht von 884,200 mg erhalten, die 200,000 mg ADD 006 enthalten.

Auf entsprechende Weise können auch Tabletten anderer Dosisstärken erhalten werden.

**Beispiel 1**

Das Beispiel betrifft die Untersuchung des Freisetzungsverhaltens erfindungsgemäßer Produkte. Insbesondere wird verdeutlicht, dass sich das Freisetzungsprofil einstellen lässt, indem erfindungsgemäße Pelletkerne mit Überzügen versehen werden, die die Freisetzung steuern, wobei die Zusammensetzung der Überzüge variiert wird.

Die Freisetzung wurde jeweils mit einer Paddle-Apparatur (USP/PH. EUR.) bei einer Rührgeschwindigkeit von 50 Umdrehungen pro Minute in Phosphatpuffer pH 6,8 als Testmedium bei einer Temperatur von 37°C bestimmt.

Zunächst wurden Pellets mit dem Wirkstoff ADD 006 hergestellt, wie es in den Herstellungsbeispielen 1 und 2 beschrieben wurde. Diese Pellets wurden dann zur Steuerung der Freisetzung überzogen. Dazu wurde eine Polyvinylacetat-Dispersion mit einem Gehalt an Talkum (10 Gew.-%) und Triethylcitrat (10 Gew.-%) verwendet. Die Dicke des Überzugs wurde variiert, indem verschiedene Mengen an Überzugsdispersion mit Polyvinylacetat sowie 10 Gew.-% Talkum und 10 Gew.-% Triethylcitrat, jeweils bezogen auf die Menge an Polyvinylacetat-Feststoff, eingesetzt wurden. Diese Mengen betrugen in den einzelnen Experimenten, bezogen auf die wirkstoffhaltigen Pelletkerne, 30 Gew.-%, 32,5 Gew.-%, 35 Gew.-%, 37,5 Gew.-% sowie 40 Gew.-%. Pellets mit einem Überzug von 40 Gew.-% der vorstehenden Polyvinylacetat-Zusammensetzung wurden außerdem mit 2 Gew.-% eines Überzugs aus Hydroxypropylmethylcellulose und kolloidalem Siliciumdioxid versehen, wobei sich letztere Gewichtsprozentangabe aus dem Gewicht der festen Bestandteile des Schutzüberzugs, bezogen auf das Gewicht der Pellets mit dem Polyvinylacetat-Überzug, errechnet.

Proben der einzelnen Ansätze wurden dann jeweils Freisetzungstests unterzogen. Die Ergebnisse sind in Fig. 1 gezeigt.

**Beispiel 2**

Das Beispiel betrifft die Untersuchung des Freisetzungsverhaltens erfindungsgemäßer Produkte. Insbesondere wird verdeutlicht, dass sich das Freisetzungsprofil einstellen lässt, indem erfindungsgemäße Pelletkerne mit Überzügen versehen werden, die die Freisetzung steuern, wobei die Zusammensetzung der Überzüge variiert wird.

Zunächst wurden Pellets mit dem Wirkstoff ADD 006 hergestellt, wie es in den Herstellungsbeispielen 1 und 2 beschrieben wurde. Diese Pellets wurden dann zur Steuerung der Freisetzung überzogen. Dazu wurde eine Polyvinylacetat-Dispersion mit einem Gehalt an Talkum (10 Gew.-%) und Triethylcitrat (7,5 Gew.-%) verwendet. Die Dicke des Überzugs wurde variiert, indem verschiedene Mengen an Überzugsdispersion mit Polyvinylacetat sowie 10 Gew.-% Talkum und 7,5 Gew.-% Triethylcitrat, jeweils bezogen auf die Menge an Polyvinylacetat-Feststoff, eingesetzt wurden. Diese Mengen betrugen in den einzelnen Experimenten, bezogen auf die wirkstoffhaltigen Pelletkerne, 30 Gew.-%, 32,5 Gew.-%, 35 Gew.-%, 37,5 Gew.-% sowie 40 Gew.-%. Pellets mit einem Überzug von 40 Gew.-% der vorstehenden Polyvinylacetat-Zusammensetzung wurden außerdem mit 2 Gew.-% eines Überzugs aus Hydroxypropylmethylcellulose und kolloidalem Siliciumdioxid versehen, wobei sich letztere Gewichtsprozentangabe aus dem Gewicht der festen Bestandteile des Schutzüberzugs, bezogen auf das Gewicht der Pellets mit dem Polyvinylacetat-Überzug, errechnet. Proben der einzelnen Ansätze wurden dann jeweils Freisetzungstests unterzogen. Die Freisetzungstests wurden durchgeführt, wie es in Beispiel 1 beschrieben ist. Die Ergebnisse sind in Fig. 2 gezeigt.

**Beispiel 3**

Das Beispiel betrifft die Untersuchung des Freisetzungsverhaltens erfindungsgemäßer Produkte. Insbesondere verdeutlicht das Beispiel den Einfluss der Tablettierung auf das Freisetzungsverhalten.

Zunächst wurden Pellets mit dem Wirkstoff ADD 006 hergestellt, wie es in den Herstellungsbeispielen 1 und 2 beschrieben wurde. Diese Pellets wurden dann zur Steuerung der Freisetzung überzogen. Dazu wurde eine Polyvinylacetat-Dispersion mit einem Gehalt an Talkum (10 Gew.-%) und Triethylcitrat (10 Gew.-%) verwendet. Die Pellets wurden mit einer Menge von 40 Gew.-% Überzugsdispersion versehen, wobei sich die Gewichtsprozentangabe aus dem Feststoffgehalt des Überzugs (Polyvinylacetat, Talkum und Triethylcitrat), bezogen auf das Gewicht der Pelletkerne, ergibt. Die Pellets wurden sodann mit 2 Gew.-% eines Überzugs aus Hydroxypropylmethylcellulose und kolloidalem Siliciumdioxid versehen, wobei sich letztere Gewichtsprozentangabe aus dem Gewicht der festen Bestandteile des Schutzüberzugs, bezogen auf das Gewicht der Pellets mit dem Polyvinylacetat-Überzug, errechnet. Das Freisetzungsverhalten dieser Pellets ist in Fig. 3 gezeigt.

Aus den Pellets wurden außerdem Tabletten hergestellt. Auch das Freisetzungsverhalten der Tabletten wurde untersucht, wie es in Beispiel 1 beschrieben ist, und ist in Fig. 3 dargestellt.

**Beispiel 4**

Das Beispiel betrifft die Untersuchung des Freisetzungsverhaltens erfindungsgemäßer Produkte. Insbesondere verdeutlicht das Beispiel den Einfluss der Tablettierung auf das Freisetzungsverhalten.

Zunächst wurden Pellets mit dem Wirkstoff ADD 006 hergestellt, wie es in den Herstellungsbeispielen 1 und 2 beschrieben wurde. Diese Pellets wurden dann zur Steuerung der Freisetzung überzogen. Dazu wurde eine Polyvinylacetat-Dispersion mit einem Gehalt an Talkum (10 Gew.-%) und Triethylcitrat (7,5 Gew.-%) verwendet. Die Pellets wurden mit einer Menge von 40 Gew.-% Überzugsdispersion versehen, wobei sich die Gewichtsprozentangabe aus dem Feststoffgehalt des Überzugs (Polyvinylacetat, Talkum und Triethylcitrat), bezogen auf das Gewicht der Pelletkerne, ergibt. Die Pellets wurden sodann mit 2 Gew.-% eines Überzugs aus Hydroxypropylmethylcellulose und kolloidalem Siliciumdioxid versehen, wobei sich letztere Gewichtsprozentangabe aus dem Gewicht der festen Bestandteile des Schutzüberzugs, bezogen auf das Gewicht der Pellets mit dem Polyvinylacetat-Überzug, errechnet. Das Freisetzungsverhalten dieser Pellets ist in Fig. 4 gezeigt.

Aus den Pellets wurden außerdem Tabletten hergestellt. Auch das Freisetzungsverhalten der Tabletten wurde untersucht, wie es in Beispiel 1 beschrieben ist, und ist in Fig. 4 dargestellt.

**Testbeispiele**

Die folgenden Beispiele betreffen die Untersuchung von Pelletkernen zur Bestimmung der Oberflächenrauheit.

**Testbeispiel 1**

Es wurden Pellets der Chargenbezeichnung SFD E 0724 untersucht. Eine elektronenmikroskopische Aufnahme von Pellets dieser Art ist in Fig. 6 gezeigt. Ein Ausschnitt aus der Oberfläche eines Pellets dieser Charge wurde mit einem Laserprofilometer abgetastet, wie es vorstehend beschrieben wurde. Eine grafische Darstellung der Messergebnisse ist in Fig. 7 A als Oberflächengrafik und in Fig. 7 B als Höhenliniendiagramm gezeigt. Zur Berechnung der Rauheit wurde ein reduzierter Datensatz verwendet, wie es vorstehend beschrieben wurde. Eine Oberflächengrafik auf der Grundlage des reduzierten Datensatzes ist in Fig. 7 C gezeigt, das entsprechende Höhenliniendiagramm in Fig. 7 D.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 385 $\mu$m; 324 $\mu$m und -293 $\mu$m bestimmt. Der optimale Radius R betrug 516 $\mu$m. Die Verteilung der Daten nach der Anpassung ist in Fig. 7 E gezeigt. In dieser Figur bezeichnet die horizontale Achse die gemessenen Datenpunkte. Die vertikale Achse bezeichnet den Abstand der gemessenen Profilpunkte von der Oberfläche der idealen Kugel mit den Mittelpunktskoordinaten und dem Radius, wie sie vorstehend angegeben wurden. Die Datenpunkte sind gleichmäßig oberhalb und unterhalb der horizontalen Achse verteilt.

Die statistische Analyse der Daten zeigt, dass die mittlere Rauheit $\sigma_d$ den Wert 13,6 $\mu$m hat und dass die relative mittlere Rauheit $\sigma_d/R$ den Wert 2,64 % hat. Die absolute Rauheit weist einen Wert von mehr als 50 $\mu$m auf. Der untersuchte Pelletkern erfüllt somit nicht die erfindungsgemäßen Kriterien für einen Pelletkern mit glatter Oberfläche.

**Testbeispiel 2**

Ein weiteres Pellet aus der gleichen Charge, die bereits im Testbeispiel 1 angesprochen wurde, wurde auf die gleiche Weise untersucht, wie es vorstehend beschrieben wurde. Eine grafische Darstellung der Messergebnisse ist in Fig. 8 A als Oberflächengrafik und in Fig. 8 B als Höhenliniendiagramm gezeigt. Der reduzierte Datensatz, der zur Berechnung der Rauheit verwendet wurde, ist die Grundlage der Oberflächengrafik, die in Fig. 8 C gezeigt ist, sowie des Höhenliniendiagramms, das in Fig. 8 D gezeigt ist.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 434 $\mu$m; 336 $\mu$m und -841 $\mu$m bestimmt. Der optimale Radius R betrug 983 $\mu$m. Die Verteilung der Daten nach der Anpassung ist in Fig. 8 E gezeigt. Die statistische Analyse der Daten zeigt, dass die mittlere Rauheit $\sigma_d$ den Wert 15,9 $\mu$m hat und dass die relative mittlere Rauheit $\sigma_d$/R den Wert 1,62 % hat. Die absolute Rauheit weist einen Wert von mehr als 50 $\mu$m auf. Der untersuchte Pelletkern erfüllt somit nicht die erfindungsgemäßen Kriterien für einen Pelletkern mit glatter Oberfläche.

**Testbeispiel 3**

Es wurden Pellets der Chargenbezeichnung SFD E 0718 untersucht. Eine elektronenmikroskopische Aufnahme von Pellets dieser Art ist in Fig. 9 gezeigt. Ein Ausschnitt aus der Oberfläche eines Pellets dieser Charge wurde mit einem Laserprofilometer abgetastet, wie es vorstehend beschrieben wurde. Eine grafische Darstellung der Messergebnisse ist in Fig. 10 A als Oberflächengrafik und in Fig. 10 B als Höhenliniendiagramm gezeigt. Zur Berechnung der Rauheit wurde ein reduzierter Datensatz verwendet, wie es vorstehend beschrieben wurde. Eine Oberflächengrafik auf der Grundlage des reduzierten Datensatzes ist in Fig. 10 C gezeigt, und das entsprechende Höhenliniendiagramm ist in Fig. 10 D dargestellt.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 391 $\mu$m; 337 $\mu$m und -680 $\mu$m bestimmt. Der optimale Radius R betrug 713 $\mu$m. Die Verteilung der Daten nach der Anpassung ist in Fig. 10 E gezeigt. Die statistische Analyse der Daten ergibt, dass die mittlere Rauheit $\sigma_d$ den Wert 10,7 $\mu$m hat und dass die relative mittlere Rauheit $\sigma_d$/R den Wert 1,5 % hat. Die absolute Rauheit weist einen Wert von etwa 50 $\mu$m auf. Der untersuchte Pelletkern erfüllt somit nicht die erfindungsgemäßen Kriterien für einen Pelletkern mit glatter Oberfläche.

**Testbeispiel 4**

Ein weiteres Pellet aus der gleichen Charge, die bereits im Testbeispiel 3 angesprochen wurde, wurde auf die gleiche Weise untersucht, wie es vorstehend beschrieben wurde. Eine grafische Darstellung der Messergebnisse ist in Fig. 11 A als Oberflächengrafik und in Fig. 11 B als Höhenliniendiagramm gezeigt. Der reduzierte Datensatz, der zur Berechnung der Rauheit verwendet wurde, ist die Grundlage der Oberflächengrafik, die in Fig. 11 C gezeigt ist, sowie des Höhenliniendiagramms, das in Fig. 11 D gezeigt ist.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 309 $\mu$m; 297 $\mu$m und -656 $\mu$m bestimmt. Der optimale Radius R betrug 804 $\mu$m. Die Verteilung der Daten nach der Anpassung ist in Fig. 11 E gezeigt. Die statistische Analyse der Daten zeigt, dass die mittlere Rauheit $\sigma_d$ den Wert 14,31 $\mu$m hat und dass die relative mittlere Rauheit $\sigma_d$/R den Wert 1,78 % hat. Die absolute Rauheit weist einen Wert von 45 $\mu$m auf. Der untersuchte Pelletkern erfüllt somit nicht die erfindungsgemäßen Kriterien von dem Pelletkern mit glatter Oberfläche.

**Testbeispiel 5**

Es wurden Pellets der Chargenbezeichnung SFD E 0572 untersucht. Eine elektronenmikroskopische Aufnahme von Pellets dieser Art ist in Fig. 12 gezeigt. Ein Ausschnitt aus der Oberfläche eines Pellets dieser Charge wurde mit einem Laserprofilometer abgetastet, wie es vorstehend beschrieben wurde. Eine grafische Darstellung der Messergebnisse ist in Fig. 13 A als Oberflächengrafik und in Fig. 13 B als Höhenliniendiagramm gezeigt. Zur Berechnung der Rauheit wurde ein reduzierter Datensatz verwendet, wie es vorstehend beschrieben wurde. Eine Oberflächengrafik auf der Grundlage des reduzierten Datensatzes ist in Fig. 13 C gezeigt, und das entsprechende Höhenliniendiagramm ist in Fig. 13 D dargestellt.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 349 $\mu$m; 315 $\mu$m und -369 $\mu$m bestimmt. Der optimale Radius R betrug 595 $\mu$m. Die Verteilung der Daten nach der Anpassung ist in Fig. 13 E gezeigt. Die statistische Analyse der Daten ergibt, dass die mittlere Rauheit $\sigma_d$ den Wert 5,5 $\mu$m hat und dass die relative mittlere Rauheit $\sigma_d$/R den Wert 0,92 % hat. Die absolute Rauheit weist einen Wert von 17 $\mu$m auf. Der untersuchte Pelletkern erfüllt somit die erfindungsgemäßen Kriterien für einen Pelletkern mit glatter Oberfläche.

**Testbeispiel 6**

Es wurden Pellets der Chargenbezeichnung SFD E 0614 untersucht. Eine elektronenmikroskopische Aufnahme von Pellets dieser Art ist in Fig. 14 gezeigt. Ein Ausschnitt aus der Oberfläche eines Pellets dieser Charge wurde mit einem Laserprofilometer abgetastet, wie es vorstehend beschrieben wurde. Eine grafische Darstellung der Messergebnisse ist in Fig. 15 A als Oberflächengrafik und in Fig. 15 B als Höhenliniendiagramm gezeigt. Zur Berechnung der Rauheit wurde ein reduzierter Datensatz verwendet, wie es vorstehend beschrieben wurde. Eine Oberflächengrafik auf der Grundlage des reduzierten Datensatzes ist in Fig. 15 C gezeigt, und das entsprechende Höhenliniendiagramm ist in Fig. 15 D dargestellt.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 293 $\mu$m; 919 $\mu$m und -358 $\mu$m bestimmt. Der optimale Radius R betrug 677 $\mu$m. Die Verteilung der Daten nach der Anpassung ist in Fig. 15 E gezeigt. Die statistische Analyse der Daten ergibt, dass die mittlere Rauheit $\sigma_d$ den Wert 7,1 $\mu$m hat und dass die relative mittlere Rauheit $\sigma_d$/R den Wert 1,06 % hat. Die absolute Rauheit weist einen Wert von 19 $\mu$m auf. Der untersuchte Pelletkern erfüllt somit die erfindungsgemäßen Kriterien für einen Pelletkern mit glatter Oberfläche.

**Testbeispiel 7**

Ein weiteres Pellet aus der gleichen Charge, die bereits im Testbeispiel 6 angesprochen wurde, wurde auf die gleiche Weise untersucht, wie es vorstehend beschrieben wurde. Eine grafische Darstellung der Messergebnisse ist in Fig. 16 A als Oberflächengrafik und in Fig. 16 B als Höhenliniendiagramm gezeigt. Der reduzierte Datensatz, der zur Berechnung der Rauheit verwendet wurde, ist die Grundlage der Oberflächengrafik, die in Fig. 16 C gezeigt ist, sowie des Höhenliniendiagramms, das in Fig. 16 D gezeigt ist.

Nach der Methode der kleinsten Fehlerquadrate wurde eine Kugel bestimmt, die optimal die Messpunkte des reduzierten Satzes wiedergibt. Die Mittelpunktskoordinaten dieser Kugel wurden zu 272 $\mu$m; 200 $\mu$m und -491 $\mu$m bestimmt. Der optimale Radius R betrug 652 $\mu$m. Die Verteilung der Daten nach der Anpassung ist in Fig. 16 E gezeigt. Die statistische Analyse der Daten zeigt, dass die mittlere Rauheit $\sigma_d$ den Wert 8,2 $\mu$m hat und dass die relative mittlere Rauheit $\sigma_d$/R den Wert 1,26 % hat. Die absolute Rauheit weist einen Wert von 30 $\mu$m auf. Der untersuchte Pelletkern erfüllt somit die erfindungsgemäßen Kriterien für einen Pelletkern mit glatter Oberfläche.

**Patentansprüche**

1. Pharmazeutisches Pellet, umfassend einen kugelförmigen wirkstoffhaltigen Kern mit glatter Oberfläche und einen Überzug auf dem Kern, der pH-unabhängig die Wirkstofffreisetzung steuert, wobei der Kern eine mittlere Rauheit von weniger als 10 $\mu$m und vorzugsweise von weniger als 7,5 $\mu$m und/oder eine relative mittlere Rauheit von weniger als 2 % aufweist.

2. Pellet nach Anspruch 1, wobei der Kern ein Länge-Breite-Verhältnis von beispielsweise weniger als etwa 1,4, vorzugsweise weniger als etwa 1,3, stärker bevorzugt weniger als etwa 1,2, noch stärker bevorzugt weniger als etwa 1,1 und insbesondere weniger als 1,05 aufweist.

3. Pellet nach Anspruch 1 oder 2, wobei der Kern einen Durchmesser im Bereich von 0,2 bis 2 mm, insbesondere von 0,4 bis 1,6 mm und ganz besonders von 0,4 bis 1,4 mm aufweist.

4. Pellet nach einem der vorstehenden Ansprüche, wobei der Überzug ein Polymer enthält, das unter Ammonium-methacrylat-copolymer, Celluloseacetatbutyrat, Celluloseacetat, Celluloseacetatpropionat, Ethylcellulose, Poly (ethylacrylat-methylacrylat) und Polyvinylacetat ausgewählt ist.

5. Pellet nach einem der vorstehenden Ansprüche, wobei zusätzlich ein äußerer Schutzüberzug vorgesehen ist, der einen wasserlöslichen Filmbildner, vorzugsweise Hydroxypropylmethylcellulose enthält und/oder wobei zwischen dem wirkstoffhaltigen Kern und dem Überzug, der die Wirkstofffreisetzung steuert, eine Zwischenschicht vorgesehen ist, die einen wasserlöslichen Filmbildner, vorzugsweise Hydroxypropylmethylcellulose, enthält.

6. Pellet nach einem der vorstehenden Ansprüche, wobei der Kern aufgebaut ist aus einem wirkstofffreien Starterkern und einer darauf befindlichen wirkstoffhaltigen Schicht, wobei der Starterkern vorzugsweise ein oder mehrere Kohlenhydrate enthält und insbesondere unter Zuckerkügelchen und Kügelchen aus mikrokristalliner Cellulose ausgewählt ist.

7. Pellet nach Anspruch 6, wobei die wirkstoffhaltige Schicht 50 % oder mehr, vorzugsweise 70 % oder mehr, an Wirkstoff enthält.

8. Pellet nach einem der Ansprüche 1 bis 5, wobei der Kern den Wirkstoff in homogener Verteilung enthält, und wobei der Kern neben dem Wirkstoff einen Träger und/oder ein Bindemittel enthält.

9. Pellet nach einem der vorstehenden Ansprüche, wobei es sich bei dem Wirkstoff um einen wasserlöslichen Wirkstoff oder ein wasserlösliches Salz eines schwer löslichen Wirkstoffs handelt.

10. Pellet nach einem der vorstehenden Ansprüche, wobei die Freisetzung des Wirkstoffs einem Profil mit einer Lag-Phase von 60 Minuten bis 840 Minuten, vorzugsweise von 60 Minuten bis 540 Minuten, folgt, wobei während der Lag-Phase ein Anteil von 5 Gew.-% oder weniger des Wirkstoffs freigesetzt wird und/oder nach einer Lag-Phase innerhalb von 60 Minuten mindestens 80 Gew.-% des noch verbliebenen Wirkstoffs freigesetzt werden.

11. Pellet nach einem der Ansprüche 1 bis 10, wobei der Wirkstoff aus dem Pellet mit einem Profil freigesetzt wird, derart, dass nach einer Lag-Phase die Freisetzung des Wirkstoffs zwischen 3 und 25 Gew.-% pro Stunde, vorzugsweise zwischen 3 und 15 Gew.-% pro Stunde, insbesondere zwischen 3 und 6,5 Gew.-% pro Stunde, beträgt.

12. Kollektiv von Pellets, wobei mindestens 90 % der Definition gemäß einem der vorstehenden Ansprüche entsprechen, wobei die Pellets vorzugsweise eine solche Teilchengrößenverteilung aufweisen, dass 90 % der Pellets einen Durchmesser haben, der vom mittleren Durchmesser um nicht mehr als den halben mittleren Durchmesser abweicht.

13. Kollektiv von Kernen, wobei mindestens 90 % die Anforderungen an die Kerne gemäß einem der Ansprüche 1 bis 3 erfüllen.

14. Verfahren zur Herstellung eines Pellets nach einem der vorstehenden Ansprüche 1 bis 11, wobei das Verfahren folgende Stufen umfasst:

(a) Bereitstellung eines wirkstoffhaltigen Pelletkerns mit einem Länge-Breite-Verhältnis von weniger als etwa 1,4 und einer mittleren Rauheit von weniger als 10 μm und/oder einer relativen mittleren Rauheit von weniger als 2 %;
(b) Aufsprühen einer wässrigen oder organischen Lösung oder Dispersion, die einen Filmbildner enthält, der die Wirkstofffreisetzung pH-unabhängig steuert.

15. Verfahren zur Herstellung einer Tablette, das folgende Stufen umfasst:

(a) Mischen von Pellets nach einem der Ansprüche 1 bis 5 mit einem oder mehreren Bestandteilen, ausgewählt unter Füllstoffen, Bindemitteln, Zerfallsmitteln, Fließreguliermitteln und Gleitmitteln, unter Bildung eines Gemisches; und
(b) Verpressen des Gemisches unter Bildung einer Tablette, wobei während der Stufe des Verpressens vorzugsweise ein Anteil der Pellets zerstört wird, so dass die Freisetzung des Wirkstoffs aus der Tablette keine Lag-Phase zeigt.

16. Tablette, herstellbar nach einem Verfahren gemäß Anspruch 15.


**Claims**

1. A pharmaceutical pellet, comprising a spherical core containing the active substance with a smooth surface and a coating on the core, which controls pH-independent release of the active substance, wherein the core has a mean roughness of less than 10 μm and preferably of less than 7.5 μm and/or a relative mean roughness of less than 2%.

2. The pellet as claimed in claim 1, wherein the core has a length-width ratio of for example less than about 1.4, preferably less than about 1.3, more preferably less than about 1.2, even more preferably less than about 1.1 and in particular less than 1.05.

3. The pellet as claimed in claim 1 or 2, wherein the core has a diameter in the range from 0.2 to 2 mm, in particular from 0.4 to 1.6 mm and quite especially from 0.4 to 1.4 mm.

4. The pellet as claimed in anyone of the preceding claims, wherein the coating contains a polymer, which is selected from ammonium-methacrylate copolymer, cellulose acetate-butyrate, cellulose acetate, cellulose acetate-propionate, ethyl cellulose, poly(ethylacrylate-methylacrylate) and polyvinyl acetate.

**5.** The pellet according to anyone of the preceding claims, wherein additionally an outer protective coating is provided, which contains a water-soluble film-forming agent, preferably hydroxypropyl methylcellulose, and/or an interlayer, which contains a water-soluble film-forming agent, preferably hydroxypropyl methylcellulose, is provided between the core containing the active substance and the coating that controls the release of the active substance.

**6.** The pellet as claimed in anyone of the preceding claims, wherein the core is constructed from a seed core not containing the active substance and a layer thereon that contains the active substance, wherein the seed core preferably contains one or more carbohydrates and in particular is selected from sugar beads and beads of microcrystalline cellulose.

**7.** The pellet as claimed in claim 6, wherein the layer containing the active substance contains 50 % or more, preferably 70 % or more, of active substance.

**8.** The pellet as claimed in anyone of claims 1 to 5, wherein the active substance is contained uniformly distributed in the core, and wherein the core contains a carrier and/or a binder in addition to the active substance.

**9.** The pellet as claimed in anyone of the preceding claims, wherein the active substance is a water-soluble active substance or a water-soluble salt of a sparingly soluble active substance.

**10.** The pellet as claimed in anyone of the preceding claims, wherein the release of the active substance follows a profile with a lag-phase from 60 minutes to 840 minutes, preferably from 60 minutes to 540 minutes, wherein during the lag-phase a proportion of 5 wt.% or less of the active substance is released and/or wherein after a lag-phase, at least 80 wt.% of the active substance still remaining is released within 60 minutes.

**11.** The pellet as claimed in anyone of claims 1 to 10, wherein the active substance is released from the pellet with a profile such that after a lag-phase, the release of the active substance is between 3 and 25 wt.% per hour, preferably between 3 and 15 wt.% per hour, in particular between 3 and 6.5 wt.% per hour.

**12.** A collection of pellets, wherein at least 90% of the pellets correspond to the definition according to anyone of the preceding claims, wherein the pellets have a particle size distribution such that 90% of the pellets have a diameter that differs from the mean diameter by not more than half the mean diameter.

**13.** A collection of cores, wherein at least 90% meet the requirements for the cores according to anyone of claims 1 to 3.

**14.** A method for the production of a pellet as claimed in anyone of the preceding claims 1 to 11, wherein the method comprises the following stages:

a) preparation of a pellet core containing the active substance, with a length-width ratio of less than about 1.4 and a mean roughness of less than 10 $\mu$m and/or a relative mean roughness of less than 2%;
b) spraying-on of an aqueous or organic solution or dispersion containing a film-forming agent that controls the pH-independent release of the active substance.

**15.** A method for the production of a tablet, comprising the following stages:

a) mixing of pellets as claimed in anyone of claims 1 to 5 with one or more ingredients, selected from fillers, binders, disintegrants, flow regulators and lubricants, with formation of a mixture; and
b) compression of the mixture with formation of a tablet, wherein during the compression stage some of the pellets are disrupted, so that the release of the active substance from the tablet does not exhibit a lag-phase.

**16.** A tablet, which can be produced according to a method as claimed in claim 15.

**Revendications**

**1.** Pellet pharmaceutique comprenant un noyau sphérique contenant un principe actif et présentant une surface lisse ainsi qu'un enrobage qui recouvre le noyau et qui régule la libération du principe actif indépendamment de la valeur pH, le noyau ayant une rugosité moyenne inférieure à 10 $\mu$m et de préférence inférieure à 7,5 $\mu$m et/ou une rugosité relative moyenne inférieure à 2 %.

**2.** Pellet selon la revendication 1, dans lequel le rapport longueur-largeur du noyau étant par exemple inférieur à 1,4 environ, de préférence inférieur à 1,3 environ, de préférence encore inférieur à 1,2 environ, de plus grande préférence encore inférieur à 1,1 environ et notamment inférieur à 1,05.

**3.** Pellet selon la revendication 1 ou 2, dans lequel le noyau présente un diamètre compris entre 0,2 et 2 mm, notamment entre 0,4 et 1,6 mm et très notamment entre 0,4 et 1,4 mm.

**4.** Pellet selon l'une des revendications précédentes, dans lequel l'enrobage contient de préférence un polymère sélectionné dans le groupe comprenant le copolymère de méthacrylate d'ammonium, le butyrate d'acétate de cellulose, l'acétate de cellulose, le propionate d'acétate de cellulose, la cellulose d'éthyle, poly(ethyl acrylate-methylacrylate) et l'acétate de polyvinyle.

**5.** Pellet selon l'une des revendications précédentes, dans lequel un enrobage extérieur de protection est par ailleurs prévu contenant un agent filmogène soluble dans l'eau, de préférence de la cellulose d'hydroxypropyl méthyle, et/ou dans lequel, entre le noyau contenant le principe actif et l'enrobage contrôlant la libération du principe actif, une couche intermédiaire est prévue qui contient un agent filmogène soluble dans l'eau, de préférence de la cellulose d'hydroxypropylméthyle.

**6.** Pellet selon l'une des revendications précédentes, dans lequel le noyau est conçu de manière à comprendre un noyau de démarrage exempt de principe actif et une couche appliquée sur celui-ci, celle-ci contenant du principe actif, le noyau de démarrage contenant, de préférence, un ou plusieurs hydrate/s de carbone et étant sélectionné notamment dans des globules de sucre et des globules en cellulose microcristalline.

**7.** Pellet selon la revendication 6, dans lequel la couche contenant le principe actif contient 50 % ou plus, de préférence 70 % ou plus de principe actif.

**8.** Pellet selon l'une des revendications 1 à 5, dans lequel le noyau contient le principe actif en distribution homogène, et le noyau contenant, à côté du principe actif, un support et/ou un agent liant.

**9.** Pellet selon l'une des revendications précédentes, dans lequel le principe actif est un principe actif soluble dans l'eau ou un sel soluble dans l'eau d'un principe actif ayant une très faible solubilité dans l'eau.

**10.** Pellet selon l'une des revendications précédentes, dans lequel la libération du principe actif suit un profil qui comprend une phase de latence durant 60 à 840 minutes, de préférence 60 à 540 minutes, au moins 5 % en poids ou moins du principe actif étant libérés pendant la phase de latence et/ou après une phase de latence, au moins 80 % en poids du principe actif restante étant libéré dans un délai de 60 minutes.

**11.** Pellet selon l'une des revendications 1 à 10, dans lequel le principe actif du pellet est libérée suivant un profil selon lequel, après la phase de latence, 3 à 25 % en poids du principe actif sont libérés du pellet par heure, de préférence 3 à 15 % en poids par heure, notamment 3 à 6,5 % en poids par heure.

**12.** Ensemble de pellets, dans lequel au moins 90 % correspondent à la définition selon l'une des revendications précédentes, les pellets présentant, de préférence, une telle répartition de granulométrie que 90 % des pellets ont un diamètre différant du diamètre moyen d'une valeur qui ne s'élève pas à plus que la moitié du diamètre moyen.

**13.** Ensemble de noyaux, dans lequel 90 % au moins répondent aux exigences formulées pour les noyaux selon l'une des revendications 1 à 3.

**14.** Procédé de préparation d'un pellet selon l'une des revendications 1 à 11, le procédé comprenant les étapes suivantes:

(a) mise à disposition d'un noyau de pellet contenant du principe actif, présentant un rapport longueur-largeur inférieure à 1,4 environ et une rugosité moyenne inférieure à 10 $\mu$m et/ou une rugosité relative moyenne inférieure à 2 %;
(b) pulvérisation d'une solution ou d'une dispersion aqueuse ou organique contenant un agent filmogène contrôlant la libération quelle que soit la valeur pH.

**15.** Procédé de préparation d'un comprimé, comprenant les étapes suivantes:

(a) mélange de pellets selon l'une des revendications 1 à 5 contenant un ou plusieurs composants, sélectionné parmi les matières de charge, les agents liants, les agents de décomposition, les agents régulateurs de fluage et les laxatifs lubrifiants, sous formation d'un mélange; et

(b) compactage du mélange sous formation d'un comprimé, une partie du pellet étant détruite pendant l'étape de compactage de préférence, de manière que la libération du principe actif depuis le comprimé ne manifeste pas de phase de latence.

16. Comprimé, susceptible d'être préparé selon un procédé selon la revendication 15.

Fig. 1

Fig. 2

–○– SDF E 0827, 40 % + 2 % fc –□– SDF E 0830, 3A –◇– SDF E 0830, 3B –△– SDF E 0830, 3C

Fig. 3

–○– SDF E 0828, 40 % + 2 % fc –□– SDF E 0845 4A –◇– SDF E 0845 4B –△– SDF E 0845 4C

Fig. 4

Fig. 5

Fig. 6

Fig. 7A

Fig. 7B

Fig. 7C

Fig. 7D

Fig. 7E

Fig. 8A

Fig. 8B

Fig. 8C

Fig. 8D

Fig. 8E

Fig. 9

Fig. 10A

Fig. 10B

Fig. 10C

Fig. 10D

Fig. 10E

Fig. 11A

Fig. 11B

Fig. 11C

Fig. 11D

Fig. 11E

Fig. 12

Fig. 13A

Fig. 13B

Fig. 13C

Fig. 13D

Fig. 13E

Fig. 14

Fig. 15A

Fig. 15B

Fig. 15C

Fig. 15D

Fig. 15E

Fig. 16A

Fig. 16B

Fig. 16C

Fig. 16D

Fig. 16E

**IN DER BESCHREIBUNG AUFGEFÜHRTE DOKUMENTE**

**In der Beschreibung aufgeführte Patentdokumente**

- EP 163836 B1
- EP 332031 B1
- EP 332929 B1
- DE 19750042 A1